(19) Europäisches Patentamt
European Patent Office
Office européen des brevets

(11) Publication number: **0 443 862 A1**

# (12) EUROPEAN PATENT APPLICATION

(21) Application number: **91301417.1**

(22) Date of filing: **22.02.91**

(51) Int. Cl.5: **C07C 211/58**, C07C 211/57, C07C 217/84, C07C 255/58, C07C 237/10, C07C 237/04, C07D 215/40, C07D 215/38, C07D 239/42, C07D 213/74, A61K 31/135

(30) Priority: **22.02.90 JP 43638/90**

(43) Date of publication of application:
**28.08.91 Bulletin 91/35**

(84) Designated Contracting States:
**AT BE CH DE DK ES FR GB GR IT LI LU NL SE**

(71) Applicant: **SUMITOMQ PHARMACEUTICALS COMPANY, LIMITED**
**2-8, Doshomachi 2-chome, Chuo-ku**
**Osaka-shi Osaka-fu(JP)**

(72) Inventor: **Antoku, Fujio**
**14-7 Mefu-2-chome**
**Takarazuka-shi(JP)**

Inventor: **Saji, Ikutaro**
**8-Q-401 Aobaoka-Minami**
**Suita-shi(JP)**
Inventor: **Ohashi, Naohito**
**6-5 Takamidai**
**Takatsuki-shi(JP)**
Inventor: **Nagata, Ryu**
**225-401, Iwakura Nakamachi**
**Sakyo-ku, Kyoto-shi(JP)**

(74) Representative: **Cresswell, Thomas Anthony et al**
**J.A. Kemp & Co. 14 South Square Gray's Inn**
**London WC1R 5LX(GB)**

## (54) Triamine derivatives and their acid-addition salts.

(57) A triamine derivative of the formula (I)

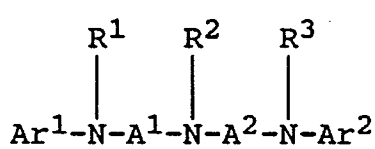

wherein $Ar^1$ and $Ar^2$ are carbocyclic or heterocyclic single ring or fused ring aromatic groups, $R^1$, $R^2$ and $R^3$ are hydrogen, lower alkyl, aryl aryl-alkyl, aryl-alkyloxycarbonyl, alkyloxycarbonyl or acyl, and $A^1$ and $A^2$ are lower alkylene groups optionally substituted by oxo, exhibits a high N-methyl-D-aspartic acid (NMDA) receptor antagonistic effect and are therefore useful as a medicine for nerve degeneration diseases.

**EP 0 443 862 A1**

FIELD OF THE INVENTION:

The present invention relates to triamine derivatives and their acid-addition salt, which are effective as glutamic acid receptor antagonists. The present invention also relates to the use of these compounds.

RELATED ART STATEMENT:

Glutamic acid is an excitatory neurotransmitter in the mammalian central nervous system. Glutaminergic nervous project from the cerebral cortex to thalamus and corpus striatum, and these means exist in cerebellum and hippocampus. In present, glutamic acid receptors are divided into three sub-type receptors, i.e., α-amino-3-hydroxy-5-methyl-4-isoxazolepropionate (AMPA) receptors, kainic acid (KA) receptors and N-methyl-D-aspartic acid (NMDA) receptors. Especially, it is known that the NMDA receptors are present only in mammals. The NMDA receptors are actually complexes of macromolecules just as the benzodiazepine receptors. The complexes consist of NMDA receptor sites, ion channel sites and some modulatory sites such as glycine sites and polyamine sites.

Antagonists, which act on NMDA receptor sites, include CPP[3-(2-carboxypiperidin-4-yl)propane-1-phosphoric acid], AP-5(2-amino-5-phosphonopentanoic acid), AP-7(2-amino-7-phosphonopentanoic acid) and the like. Antagonists at channel sites include MK-801(D-5-methyl-10, 11-dihydro-5H-dibenzo[a,d]-cycloheptene-5,10-imine), etc. Antagonists against glycine modulatory sites include 7-chloro-kynurenic acid. Antagonists at polyamine modulatory sites include diethylenetriamine.

Recently, various researches have been made on physiological function of glutamic acid as excitatory neurotransmitter and also on the relationship between glutamic acid receptors and diseases. It has been suggested that excessive stimulation of NMDA receptors may lead to celldeath in brain ischemia after stroke, hypoxia, hypoglycemia, or carbon monoxide-poisoning. Furthermore, neurodegenerative diseases such as dementia of the Alzheimer's disease, chorea, spinalcord/cerebellum degenerative disorders, apoplexy, cerebralpalsy, and epilepsy may be due to hyperactivation of NMDA receptors.

SUMMARY OF THE INVENTION:

It is an object of the invention to provide a triamine derivative which has an antagonistic action against glutamic acid causing nerve degenerative disorders with less substantial side effects.

It has been found that the triamine derivative of the general formula (I) is useful as a NMDA receptor antagonist. For example, the NMDA receptor antagonist is useful for preventing the prognostic symptoms by cerebral apoplexy and cerebral infarction. Thus, the present invention provides a NMDA receptor antagonist, which comprise the triamine derivative or its acid-addition salt of the formula:

$$Ar^1-\underset{\displaystyle R^1}{\overset{|}{N}}-A^1-\underset{}{N}(R^2)-A^2-N(R^3)-Ar^2 \qquad (I)$$

wherein $Ar^1$ represents an unsubstituted or substituted aryl group; an unsubstituted or substituted 6-membered heterocyclic aromatic group containing 1 to 3 nitrogen atoms; an unsubstituted or substituted bicyclic heterocyclic aromatic group having a 5-membered heterocyclic ring condensed with a benzene ring and containing 1 to 3 nitrogen atoms; an unsubstituted or substituted bicyclic heterocyclic aromatic group having a 5-membered heterocyclic ring condensed with a benzene ring and containing one sulfur atom and optionally one nitrogen atom; an unsubstituted or substituted bicyclic heterocyclic aromatic group having a 5-membered heterocyclic ring condensed with a benzene ring and containing one oxygen atom and optionally one nitrogen atom; an unsubstituted or substituted bicyclic carbocyclic aromatic group having a 5- or 6-membered hydrated carbocyclic ring condensed with a benzene ring; or an unsubstituted or substituted bicyclic heterocyclic aromatic group having a 6-membered heterocyclic ring condensed with a benzene ring and containing 1 or 2 nitrogen atoms;

$Ar^2$ represents an unsubstituted or substituted naphthyl group; an unsubstituted or substituted bicyclic heterocyclic aromatic group having a 5-membered heterocyclic ring condensed with a benzene ring and containing 1 to 3 nitrogen atoms; an unsubstituted or substituted bicyclic heterocyclic aromatic group having a 5-membered heterocyclic ring condensed with a benzene ring and containing one sulfur atom and optionally one nitrogen atom; an unsubstituted or substituted bicyclic heterocyclic aromatic group having a

5-membered heterocyclic ring condensed with a benzene ring and containing one oxygen atom and optionally one nitrogen atom; an unsubstituted or substituted bicyclic carbocyclic aromatic group having a 5- or 6-membered hydrated carbocyclic ring condensed with a benzene ring; or an unsubstituted or substituted bicyclic heterocyclic aromatic group having a 6-membered heterocyclic ring condensed with a benzene ring and containing 1 or 2 nitrogen atoms;

each of $A^1$ and $A^2$ independently represents a lower alkylene group optionally substituted by 1 or 2 oxo group;

each of $R^1$, $R^2$ and $R^3$ independently represents hydrogen, lower alkyl, aryl, arylalkyl, arylalkyloxycarbonyl, alkyloxycarbonyl or acyl.

Some of the compounds having the general formula (I) and their acid-addition salts are novel. Namely, the present invention also provides the novel compounds and their acid addition salts having the general formula (I), wherein $Ar^1$ represents an unsubstituted or substituted aryl group; an unsubstituted or substituted 6-membered heterocyclic aromatic group containing 1 to 3 nitrogen atoms; an unsubstituted or substituted bicyclic heterocyclic aromatic group having a 5-membered heterocyclic ring condensed with a benzene ring and containing 1 to 3 nitrogen atoms; an unsubstituted or substituted bicyclic carbocyclic aromatic group having a 5- or 6-membered hydrated carbocyclic ring condensed with a benzene ring; or an unsubstituted or substituted bicyclic heterocyclic aromatic group having a 6-membered heterocyclic ring condensed with a benzene ring and containing 1 or 2 nitrogen atoms;

$Ar^2$ represents an unsubstituted or substituted naphthyl group; an unsubstituted or substituted bicyclic heterocyclic aromatic group having a 5-membered heterocyclic ring condensed with a benzene ring and containing 1 to 3 nitrogen atoms; an unsubstituted or substituted bicyclic carbocyclic aromatic group having a 5- or 6-membered hydrated carbocyclic ring condensed with a benzene ring; or an unsubstituted or substituted bicyclic heterocyclic aromatic group having a 6-membered heterocyclic ring condensed with a benzene ring and containing 1 or 2 nitrogen atoms, with the proviso that when $Ar^1$ is an 8-quinolyl group, when both $A^1$ and $A^2$ are an ethylene group, and when $R^1$, $R^2$ and $R^3$ are hydrogen, then $Ar^2$ does not represent an 8-quinolyl group;

each of $A^1$ and $A^2$ independently represents a lower alkylene group, with the proviso that one of $A^1$ and $A^2$ represents an ethylene group optionally substituted by an alkyl group;

each of $R^1$ and $R^3$ independently represents hydrogen, lower alkyl, aryl, arylalkyl, arylalkyloxycarbonyl, alkyloxycarbonyl or acyl; and

$R^2$ represents hydrogen, lower alkyl, arylalkyl, arylalkyloxycarbonyl, alkyloxycarbonyl or acyl.

## DETAILED DESCRIPTION OF THE INVENTION

Preferably, aryl in the general formula has less than 10 carbon atoms. Examples of aryl are phenyl, naphthyl and the like. As examples of 6-membered heterocyclic aromatic groups having 1 to 3 nitrogen atoms, there may be mentioned pyridyl, pyrimidinyl, pyrazinyl, pyridazinyl, triazinyl and the like. Bicyclic heterocyclic aromatic groups having a 5-membered heterocyclic ring condensed with a benzene ring and containing 1 to 3 nitrogen atoms include indolyl, iso-indolyl, 1H-indazolyl, 1H-benzotriazolyl, etc.

As examples of bicyclic heterocyclic aromatic groups having a 5-membered heterocyclic ring condensed with a benzene ring and containing one sulfur atom and optionally one nitrogen atom, there may be mentioned benzothienyl, benzothiazolyl, benzisothiazolyl and the like.

Examples of bicyclic heterocyclic aromatic groups having a 5-membered heterocyclic ring condensed with a benzene ring and containing one oxygen atom and optionally one nitrogen atom include benzofuryl, benzisoxazolyl, benzoxazolyl and the like.

Examples of bicyclic carbocyclic aromatic groups, having a 5- or 6-membered hydrated carbocyclic ring condensed with a benzene ring include tetrahydronaphthyl and the like.

Examples of heterocyclic aromatic groups having a 6-membered heterocyclic ring condensed with a benzene ring and containing 1 or 2 nitrogen atoms include quinolyl, isoquinolyl, quinoxalyl, quinazolyl, phthalazinyl and the like.

As for aryl groups, 6-membered heterocyclic aromatic groups containing 1 to 3 nitrogen atoms, bicyclic heterocyclic aromatic groups having 5-membered heterocyclic ring condensed with a benzene ring and containing 1 to 3 nitrogen atoms, bicyclic heterocyclic aromatic groups having a 5-membered heterocyclic ring condensed with a benzene ring and containing one sulfur atom and optionally one nitrogen atom, bicyclic heterocyclic aromatic groups having a 5-membered heterocyclic ring condensed with a benzene ring and containing one oxygen atom and optionally one nitrogen atom, bicyclic carbocyclic aromatic groups having a 5- or 6-membered hydrated carbocyclic ring condensed with a benzene ring, or bicyclic heterocyclic aromatic groups having a 6-membered heterocyclic ring and containing 1 or 2 nitrogen atoms,

these groups may optionally have substituents including lower alkyl, lower alkoxy, halogen, nitro, cyano, trifluoromethyl, hydroxy, carboxyl, esterified carboxyl, amino, mercapto, hydroxyiminomethyl, acetamido, guanidyl, methanesulfinyl, methanesulfonyl, sulfamoyl, carbamoyl, N-hydroxycarbamoyl, N-hydroxyacetamido, and the like.

As examples of lower alkylene groups, there may be mentioned alkylene groups with 2 to 4 carbon atoms, including ethylene, propylene, 1-methyl-ethylene, 2-methyl-ethylene, 1,2-dimethyl-ethylene, 1-ethyl-ethylene, 2-ethyl-ethylene, 1-methyl-propylene, 2-methyl-propylene, 3-methyl-propylene and the like.

As examples of lower alkylene group substituted by 1 or 2 oxo groups may be mentioned alkylene group with 2 to 4 carbon atoms substituted by 1 or 2 oxo groups, including 1-oxoethylene, 2-oxoethylene, 1-oxopropylene, 3-oxopropylene, 1-oxo-2-methylethylene, 2-oxo-1-methylethylene, 1-oxo-2-ethylethylene, 2-oxo-1-ethylethylene, 1-oxo-2-methylpropylene, 1-oxo-3-methylpropylene 3-oxo-2-methylpropylene, 3-oxo-1-methylpropylene, 1,2-dioxoethylene, 1,3-dioxoethylene.

Lower alkyl groups may be alkyl having less than 6 carbon atoms, such as methyl, ethyl, propyl, butyl, pentyl, hexyl, 1-methylethyl, 1-methylpropyl, 2-methylpropyl, 1-ethylpropyl, 2-ethylpropyl, 1-methylbutyl, 2-methylbutyl, 3-methylbutyl, 1-ethylbutyl, 2-ethylbutyl, 3-ethylbutyl, 1-methylpentyl, 2-methylpentyl, 3-methylpentyl, 4-methylpentyl and the like. Acyl groups include aromatic acyl containing less than 11 carbon atoms or alkanoyl groups containing less than 7 carbon atoms. Example of aromatic acyl are benzoyl, and lower alkanoyl, etc. Examples of lower alkanoyl groups are acetyl, propanoyl, butanoyl, pentanoyl, hexanoyl and the like. Lower alkoxy groups may be alkoxy having less than 4 carbon atoms, including, for instance, methoxy, ethoxy, propoxy, butoxy, etc. Halogen atoms include, for instance, fluorine, chlorine, bromine and iodine atoms. Examples of esterified carboxyl groups are carboxyl esterified with lower alkyl.

As examples of arylalkyl groups, there may be mentioned benzyl, 3,4-dimethoxybenzyl, o-nitrobenzyl, di(p-methoxyphenyl)methyl, diphenyl-4-pyridylmethyl and the like. Examples of aryloxycarbonyl are benzyloxycarbonyl, 2,4,6-trimethylbenzyloxycarbonyl, p-methoxybenzyloxycarbonyl, 3,5-dimethoxybenzyloxycarbonyl, p-decyloxybenzyloxycarbonyl, p-nitrobenzyloxycarbonyl, o-nitrobenzyloxycarbonyl, 3,4-dimethoxy-6-nitrobenzyloxycarbonyl, p-bromobenzyloxycarbonyl, chlorobenzyloxycarbonyl, 2,4-dichlorobenzyloxycarbonyl, p-cyanobenzyloxycarbonyl and the like. Alkyloxycarbonyl groups include, for instance, oxycarbonyl substituted by lower alkyl. Acids, which is employed in the preparation of acid-addition salts, include inorganic acids such as hydrochloric acid, hydrobromic acid, hydriodic acid, sulfuric acid, etc., and organic acids such as acetic acid, oxalic acid, citric acid, malic acid, tartaric acid, fumaric acid, maleic acid, etc.

Depending on substituents, the compound according to the invention may sometimes have asymmetric carbon atoms, so that such an asymmetric compound will have optical or geometrical isomers. The present patent invention also includes such isomers alone and isomeric mixtures.

The glutamic acid receptor antagonist according to the invention may be administered orally or parenterally. For instance, the antagonist may be administered orally in the form of tablets, capsules, syrups, suspensions, etc. Also, the antagonist may be formulated into compositions for injections, including solutions, emulsions, suspensions and the like, or into rectal suppositories. These formulations may be prepared in a conventional manner by mixing the active ingredient with carriers, excipients, binders, stabilizers and the like. In the case of compositions for injections, it may be also possible to employ buffers, solubilizing agents, adjuvants for isotonicity, and the like.

The dose of the active ingredient will vary depending on diseases, symptoms, ages, body weights of patients, and also on the dosage form. In the case of oral dosages, the active ingredient according to the invention may usually be administered to an adult patient in an amount of 1 to 1000 mg per day. In the case of parenteral dosage, the active ingredient may, for instance, be administered one time or several times in a day in an amount of 0.1 to 500 mg.

The compound according to the invention can be prepared by the following methods.

Preparation method 1

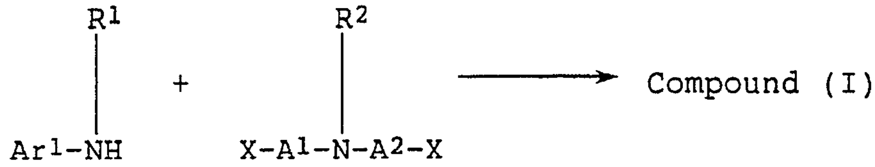


(II) (III)

In the above formulas, X represents halogen, alkyl- or aryl-sulfonyloxy such as methanesulfonyloxy, p-toluenesulfonyloxy or the like, hydroxy or lower alkoxy, and $Ar^1$, $R^1$, $R^2$, $A^1$ and $A^2$ have the same meanings as stated above. According to the preparation method 1, the compound (I) is obtained wherein $Ar^2$ is the same as $Ar^1$.

The starting compound (II) is reacted with the starting compound (III) optionally in an inert organic solvent at a temperature of -30 °C to room temperature or heating, optionally in the presence of a base or a condensing agent to produce the compound (I). As inert organic solvents, there may be mentioned, for instance, alcohols such as n-butyl alcohol, iso-propyl alcohol and the like, aromatic hydrocarbons such as benzene, toluene, xylene and the like, ethers such as diethyl ether, tetrahydrofuran and the like, pyridine, dimethylformamide, dimethylsulfoxide, acetonitrile, etc. As bases, it may be possible to use inorganic bases, including alkali metals such as lithium, sodium, potassium and the like, alkali metal hydrides such as sodium hydride, potassium hydride, etc., carbonates of alkali or alkaline earth metals such as potassium carbonate, potassium bicarbonate and the like; and organic bases including tertiary amines such as triethylamine, pyridine, dimethylaminopyridine and the like, alkyl lithiums such as butyl lithium, etc. Examples of condensing agents are dicyclohexylcarbodiimide, 1-ethyl-3-(3-dimethylaminopropyl)-carbodiimide hydrochloride, etc.

Preparation method 2

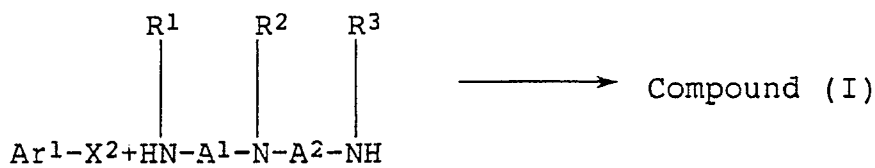


(IV) (V)

In the formulas, $X^2$ represents halogen, and $Ar^1$, $R^1$, $R^2$, $R^3$, $A^1$ and $A^2$ have the same meanings as stated above. According to the preparation method 2, the compound (I) is also obtained wherein $Ar^2$ is the same as $Ar^1$.

The starting compound (IV) is reacted with the starting compound (V) optionally in an inert solvent, optionally in the presence of a base at a temperature of -30 °C to room temperature or heating to produce the compound (I). As inert organic solvents, there may be mentioned, for instance, alcohols such as n-butyl alcohol, iso-propyl alcohol and the like, aromatic hydrocarbons such as benzene, toluene, xylene and the like, ethers such as diethyl ether, tetrahydrofuran, etc., pyridine, dimethylformamide, dimethylsulfoxide, acetonitrile and the like. As bases, use may be made, for instance, of inorganic bases including alkali metals such as lithium, sodium, potassium, etc., alkali metal hydrides such as sodium hydride, potassium hydride and the like, carbonates of alkali or alkaline earth metals such as potassium carbonate, potassium bicarbonate and the like; and organic bases including tertiary amines such as triethylamine, pyridine, dimethylaminopyridine and the like, and alkyl lithiums such as butyl lithium, etc.

Preparation method 3

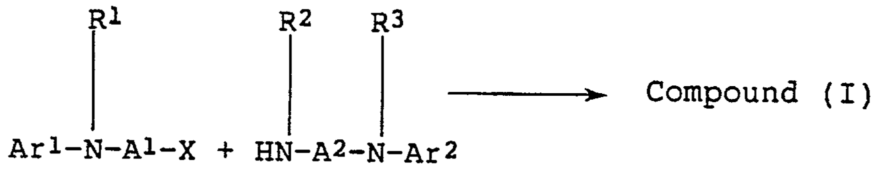


(VI) (VII)

In the formulas, $Ar^1$, $Ar^2$, $R^1$, $R^2$, $R^3$, $A^1$, $A^2$ and X have the same meanings as stated above.

The starting compound (VI) is reacted with the starting compound (VII) optionally in an inert organic solvent, optionally in the presence of a base or a condensing agent at a temperature of -30° C to room temperature or heating to produce the compound (I). As examples of inert organic solvents, there may be mentioned alcohols n-butyl alcohol, iso-propyl alcohol and the like, aromatic hydrocarbons such as benzene, toluene, xylene and the like, ethers such as diethyl ether, tetrahydrofuran and the like, pyridine, dimethylformamide, dimethylsulfoxide, acetonitrile, etc. As bases, it may be possible to use, for instance, inorganic bases including alkali metals such as lithium, sodium, potassium and the like, alkali metal hydrides such as sodium hydride, potassium hydride and the like, carbonates of alkali or alkaline earth metals such as potassium carbonate, potassium bicarbonate and the like; and organic bases including tertiary amines such as triethylamine, pyridine, dimethylaminopyridine and the like, alkyl lithiums such as butyl lithium, etc. Examples of condensing agents are dicyclohexylcarbodiimide, 1-ethyl-3-(3-dimethylaminopropyl)-carbodiimide hydrochloride and the like.

Preparation method 4

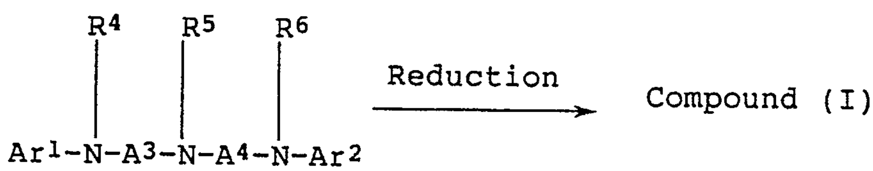


(VIII)

In the formulas, each of $A^3$ and $A^4$ independently represents lower alkylene substituted with one or two oxo group(s); each of $R^4$, $R^5$ and $R^6$ independently represents hydrogen or lower alkyl; and $Ar^1$ and $Ar^2$ have the same meanings as stated above. According to the preparation method 4, the compound (I) is obtained wherein at least one of $A^1$ and $A^2$ is a lower alkylene group.

The starting compound (VIII) is subjected to a reaction operation in an inert organic solvent in the presence of a reducing agent at a temperature of -70° C to room temperature or heating to produce the compound (I). Examples of inert organic solvents include ethers such as diethyl ether, tetrahydrofuran and the like. As reducing agents, there may be mentioned, for instance, lithium aluminium hydride, sodium aluminium hydride, sodium aluminium dialkoxy hydride such as sodium bis-2-methoxy ethoxy aluminium hydride, sodium borohydride and diborane.

Preparation method 5

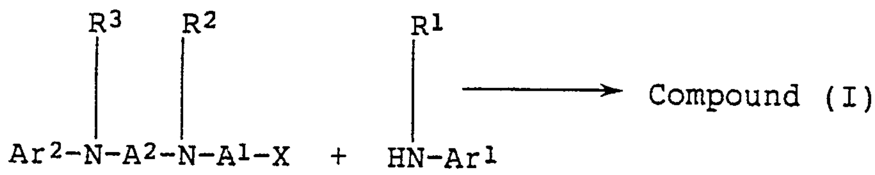


(IX) (II)

In the formulas, $Ar^1$, $Ar^2$, $R^1$, $R^2$, $R^3$, $A^1$, $A^2$ and X have the same meanings as stated above.

The starting compound (IX) is reacted with the starting compound (II) optionally in an inert organic solvent, optionally in the presence of a base or a condensing agent at a temperature of -30 °C to room temperature or heating to produce the compound (I). As inert organic solvents, it may be possible to use, for instance, alcohols such as n-butyl alcohol, iso-propyl alcohol and the like, aromatic hydrocarbons such as benzene, toluene, xylene and the like, ethers such as diethyl ether, tetrahydrofuran and the like, pyridine, dimethylformamide, dimethylsulfoxide, acetonitrile, etc. Examples of bases are inorganic bases including alkali metals such as lithium, sodium, potassium and the like, alkali metal hydrides such as sodium hydride, potassium hydride and the like, and carbonates of alkali metals or alkaline earth metals such as potassium carbonate, potassium bicarbonate and the like; and organic bases including tertiary amines such as triethylamine, pyridine, dimethylaminopyridine and the like, and alkyl lithiums such as butyl lithium, etc. As condensing agents, use may be made, for instance, of dicyclohexyl carbodiimide, 1-ethyl-3-(3-dimethylaminopropyl)-carbodiimide hydrochloride and the like.

The starting compounds (II), (III), (IV), (V), (VI), (VII), (VIII) and (IX), which are employed in the processes according to the invention, are known compounds or can be prepared by a method similar to a known synthesis method. The starting compounds (VIII) may also be prepared by a method similar to any of the above preparation methods 1, 2 and 3.

(1) Starting compound (II)

The compound (II) is known from, for example, the following publication, and commercially available as chemical reagents. Any of the compounds (II) can be prepared by a method similar to a known general synthesis method.

| Compound | Literature |
| --- | --- |
| 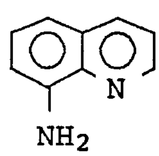 | Beil, 22, 450 |

(2) Starting compound (III)

The compound (III) is known from, for example, the following literatures, and commercially available as chemical reagent. Any of the compounds (III) can be prepared by a method similar to a known general synthesis method.

| Compound | Literature |
|---|---|
| 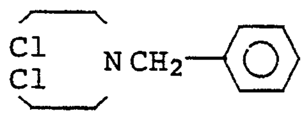 | USP 4,748,276 |
| 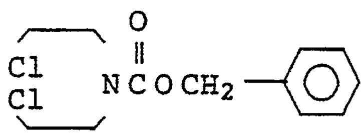 | Acta, chem. Scand. Ser. B, B33 (8), 584-586 (1979) |

(3) Starting compound (IV)

The compound (IV) is known from, for example, the following literature, and commercially available as chemical reagent. Any of the compounds (IV) can be prepared by a method similar to a known general synthesis method.

| Compound | Literature |
|---|---|
| 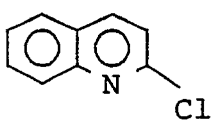 | Beil, 20, 359 |

(4) Starting compound (V)

The compound (V) is known from, for example, the following literature, and commercially available, as chemical reagent. Any of the compounds (V) can be prepared by a method similar to a known general synthesis method.

| Compound | Literature |
|---|---|
| $H_2NCH_2CH_2NH-CH_2CH_2NH_2$ | Beil, 4, 255 |

(5) Starting compound (VI)

The compound (VI) is known from, for example, the following publications and commercially available as chemical reagent. Any of the compounds (VI) can be prepared by a method similar to a known general synthesis method.

| Compound | Literature |
|---|---|
| 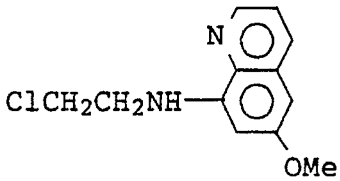 | J. Am. Chem. Soc., 81, 3984 (1959) |
| 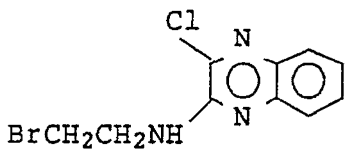 | CA 57: 12479f |
| 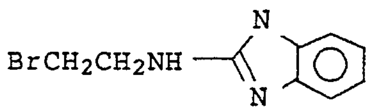 | SU 952,847 (1982) |
| 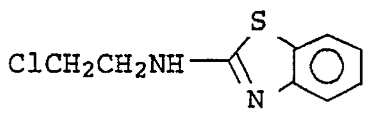 | DE 2845250 (1980) |
| 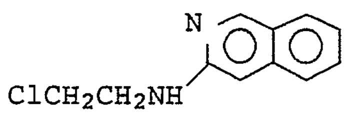 | J. Med. Chem. 16 (6) 633-7 (1973) |
| 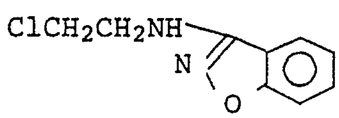 | Boll. Soc. Ital. Biol. Sper 48 (11) 297-9 (1972) |
| 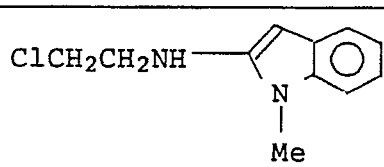 | Tetrahedron 27(4) 775-87 (1971) |
| 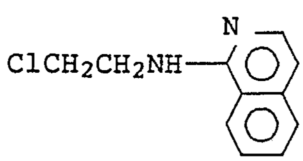 | DE 1911519 |

- Cont'd -

(Cont'd)

| | |
|---|---|
| 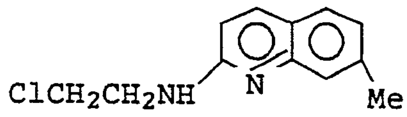 | Yakugaku Zasshi 89 (6) 767-70 (1969) |
| 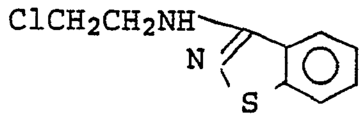 | Chem. Ber. 102 (6) 1961-75 (1969) |
| 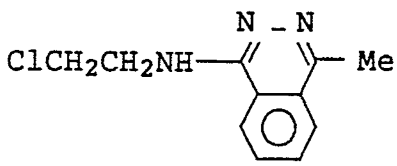 | Indian. J. Chem. 43, 308 (1966) |
| 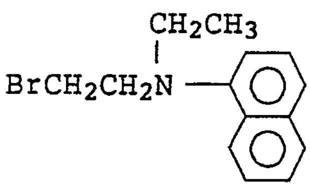 | Can. J. Physiol. Pharmacol. 54 (3) 386-92 (1972) |
| 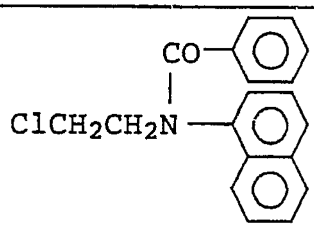 | Ann. Chem. 716, 127-34 (1968) |
| 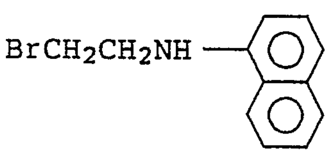 | J. Med. Chem. 6 (6) 637 (1963) |

(6) Starting compound (VII)

The compound (VII) is known from, for example, the following mentioned publications and commercially available as chemical reagent. Any of the compounds (VII) can be prepared by a method similar to a known general synthesis method.

| Compound | Literature |
| --- | --- |
| 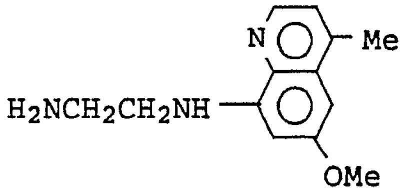 | Yiyao Gongye 17 (5) 209-13 (1986) |
| 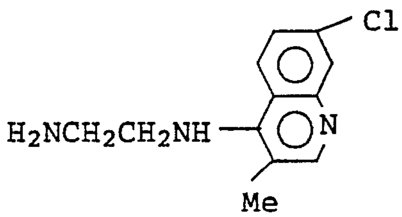 | J. Am. Chem. Soc. 81, 3984 (1959) |
| 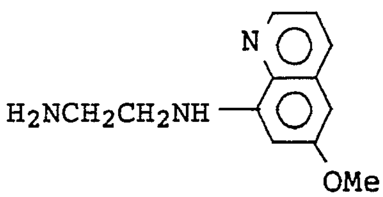 | Indian J. Chem. Sect B, 24 B (4) 419-423 (1985) |
| 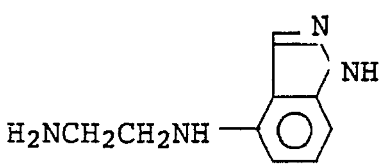 | USP 4507488 |
| 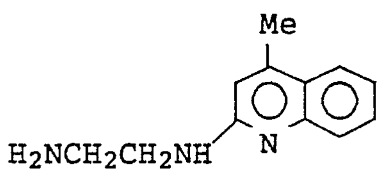 | CA 61: 6990e |
| 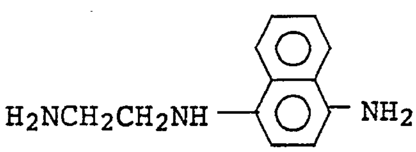 | Arch. Pharm. 316 (10) 889-91 (1983) |

- Cont'd -

(Cont'd)

| | |
|---|---|
| 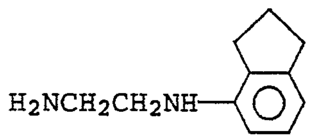 | EP 71950 (1983) |
| 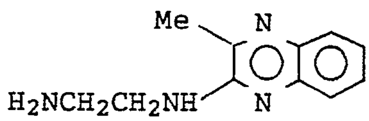 | USP 4608383 (1986)<br>EP 42593 (1981) |
| 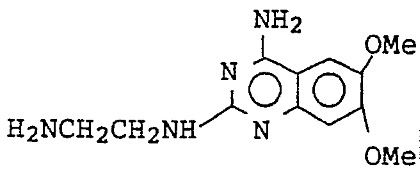 | " |
| 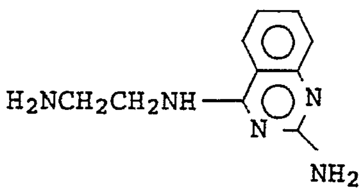 | " |
| 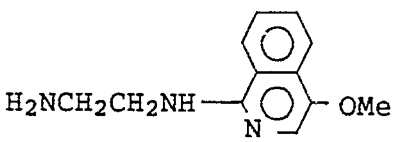 | " |
| 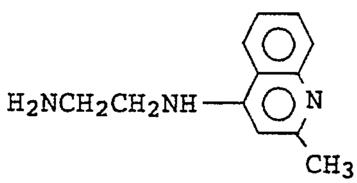 | " |
| 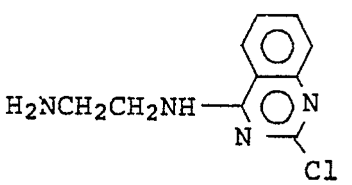 | " |

- Cont'd -

| | |
|---|---|
| 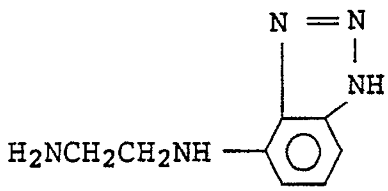 | USP 4608383 (1986)<br>USP 4507488 (1985) |
| 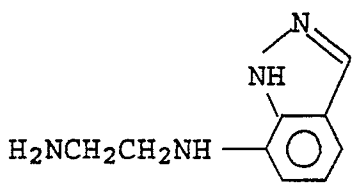 | " |
| 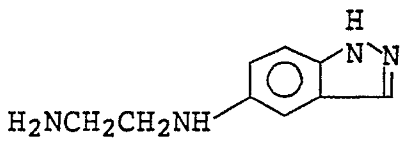 | " |
| 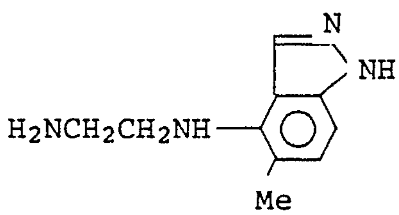 | " |
| 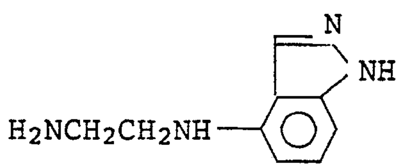 | " |
| 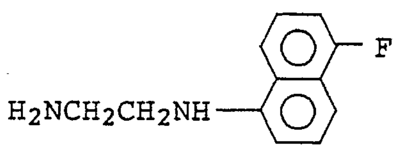 | EP 5658 (1979) |
| | |

- Cont'd -

(Cont'd)

| | |
|---|---|
| 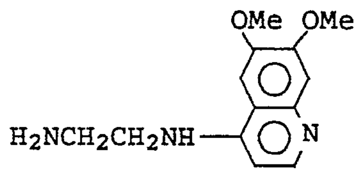 | J. Med. Chem. 14 (11) 1060-1066 (1971) |
| 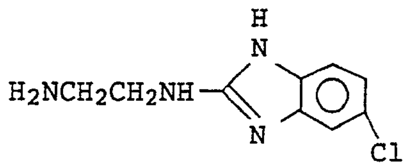 | Helv. Chim. Acta. 54 (7) 2114-2121 (1971) |
| 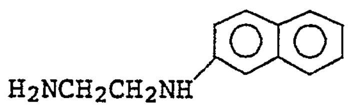 | DE 2444 835 (1975)<br>J. Med. Chem. 12 (2) 207-211 (1969) |
| 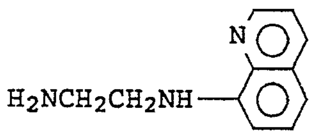 | J. Indian. Chem. Sec. 44 (2) 119-122 (1967) |
| 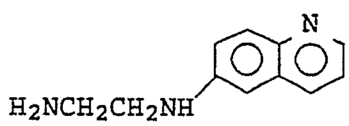 | " |
| 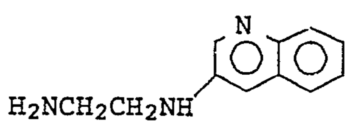 | " |
| 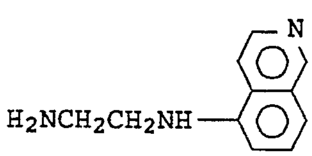 | " |

- Cont'd -

(Cont'd)

| | |
|---|---|
| 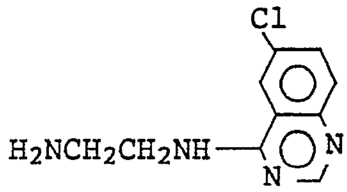 | J. Am. Chem. Soc. 71, 1871 (1949) |
| 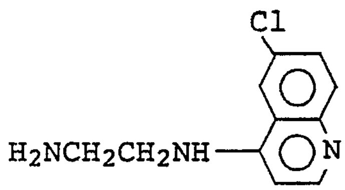 | J. Clin. Invest. 54 (1) 24-33 (1974) |

(7) Starting compound (VIII)

The compound (VIII) can be prepared by a method similar to any of the above preparation methods 1, 2, 3 and 5.

(8) Starting compound (IX)

The starting compound (IX) can be prepared by the following reaction scheme.

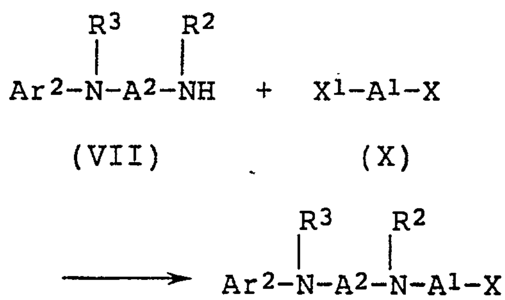

(VII) (X)

(IX)

In the formulas, $X^1$ represents a leaving group, and $Ar^2$, $R^2$, $R^3$, $A^1$, $A^2$ and X have the same meanings as stated above.

The compound (VII) is reacted with the compound (X), optionally in an inert organic solvent, optionally in the presence of a base or a condensing agent at a temperature of -30 °C to room temperature or heating to produce the starting compound (IX). As examples of inert organic solvents, there may be mentioned, for instance, alcohols such as n-butyl alcohol isopropyl alcohol and the like, aromatic hydrocarbons such as benzene, toluene, xylene and the like, ethers such as diethyl ether, tetrahydrofuran and the like, pyridine, dimethylformamide, dimethylsulfoxide, acetonitrile, etc. As bases, use may be made, for instance, of inorganic bases including alkali metals such as lithium, sodium, potassium and the like, alkali metal hydrides such as sodium hydride, potassium hydride and the like, carbonates of alkali metals or alkaline earth metals such as potassium carbonate, potassium bicarbonate and the like; and organic babes including tertiary amines such as triethylamine, pyridine, dimethylaminopyridine and the like, and alkyl lithiums such as butyl lithium, etc. Examples of condensing agents include dicyclohexyl-carbodiimide, 1-ethyl-3-(3-dimethylamino-propyl)-carbodiimide hydrochloride and the like.

Pharmacological tests, Reference Examples and Examples

The invention will be illustrated in more detailed by the Pharmacological tests, Reference Examples and Examples. However, it should be noted that the scope of the invention is not limited only to that of these Examples.

A. Pharmacological tests

(1) Binding inhibition at various sites (in vitro)

The NMDA-antagonistic activity of the triamine derivatives according to the invention can be determined by a method, wherein a measurement is made about the activity of removing [$^3$H]MK-801 from its binding sites (the activated NMDA receptor complex ion channels). Furthermore, from the below-mentioned binding inhibition experiments using radioactive ligands, it is possible to determine the sites of NMDA receptor complexes, where the antagonists have acted.

The properties of a competitive antagonists agent, which act on glutamic acid or NMDA receptor sites, can be estimated by a method, wherein a measurement is made about the capability of removing the ($^3$H)-Glu(glutamic acid) or ($^3$H)Cpp [3-(2-carboxy-piperidine-4-yl)propane-1-phosphoric acid] from its binding sites. As for the properties of a chemical compound as an antagonist inhibitor on glycine regulation sites or on polyamine regulation sites, it is possible to estimate such properties by a method, wherein a determination is made about the capability of removing ($^3$H)Gly(Glycine) or ($^3$H)SPD(spermidine) from the sites in question.

These activities can be expressed in term of $IC_{50}$ value, which is defined as the concentration ($\mu$M) of a test compound required for removing 50% of ($^3$H)MK-801, ($^3$H)Glu, (3H)Cpp, ($^3$H)Gly or ($^3$H)SPP bound.

The test methods are as follows.

## [$^3$H]MK-801 binding test

A crude sample of synapse membrane, which had been obtained from a whole brain of rat, was washed three times with a cleaning operation wherein the sample was centrifuged at 50,000 g in a 50 mM tris acetate buffer solution (pH 7.4) for 30 minutes. The pellet was suspended in a 0.32 M sucrose solution, and the resulting suspension was stored in a frozen state at -80 °C.

Before the use, the frozen suspension was melted at room temperature, and pretreated with a 0.08% triton X-100 at 2 °C for 10 minutes. Then the suspension sample was washed twice with a centrifugal cleaning operation as mentioned above, and thereafter the sample was washed and subjected to a binding test.

In the binding test, the membrane sample (about 250 $\mu$g of proteins) was incubated with 5nM [$^3$H]MK-801 (29.4Ci/mmol) at 30 °C for 30 minutes. This reaction was stopped by a suction filtration method using a Wattman GF/B glass filter. The radioactivity on the filter was determined by a liquid scintillation method. The non-specific bindings were calculated out from the radioactivity in the presence of 0.1 mM MK-801.

## [$^3$H]Glu binding test

A crude synapse membrane sample, which had been obtained from a whole brain of rat, was suspended in a 50 mM tris-acetate buffer solution (pH 7.4). The resulting suspension was centrifuged at 50,000 g for 30 minutes. This cleaning operation was three times repeated. The pellet was then suspended in a 0.32 M sucrose solution and stored as a frozen suspension at -80 °C.

The frozen suspension was melted at room temperature, and pretreated with a 0.08% triton X-100 at 2 °C for 10 minutes. Thereafter, a cleaning operation was twice carried out using centrifugal means. In the [$^3$H]Glu binding test, the membrane sample (about 100 $\mu$g of proteins) was incubated 10nM [$^3$H]Glu (30 Ci/mmol) in a 50 mM tris acetate buffer solution (pH 7.4) at 2 °C for 10 minutes. This reaction was stopped by a suction filtration method using a Wattman GF/B glass filter. The NMDA sensitive bindings were calculated out by subtracting the NMDA non-sensitive bindings (i.e., bindings in the presence of NMDA) from the total bindings.

## [3H]CPP binding test

A crude synapse membrane sample, which had been obtained from a whole brain of rat, was washed three times with a cleaning operation employing centrifugal means at 50,000 g for 30 minutes. The pellet thus obtained was suspended in a 0.32 M sucrose solution and stored in a frozen state at -80° C.

Before the use, the frozen suspension was melted at room temperature, and pretreated with a 0.08% triton X-100 at 2° C for 10 minutes. The pretreated sample was carried out twice with cleaning operation as mentioned above and then subjected to a binding test. In this test, the suspension (protein content: about 250 µg) was incubated with 10nM [$^3$H]CPP(30.7 Ci/mmol) at 2° C for 10 minutes. The reaction was stopped by a suction filtration method using a Wattman GF/B glass filter. The non-specific bindings were calculated out from the radioactivity in the presence of 1 mM Glu.

## [3H]Gly binding test

A crude synapse membrane sample, which had been obtained from a whole brain of rat, was washed three times with a centrifugal operation at 50,000 g for 30 minutes in a 50 mM tris acetate buffer solution (pH 7.4). The pellet was suspended in a 0.32 M sucrose solution, and stored in a frozen state at -80° C.

Before the use, the frozen sample was melted it room temperature, pretreated with a 0.08% triton X-100 at 2° C for 10 minutes, and then a centrifugal operation was carried out twice as mentioned above. In the test, the synapse membrane sample (protein content: about 150-200 µg) was incubated with 10 nM [$^3$H]Gly-(40 Ci/mmol) in the above-mentioned buffer solution at 2° C for 10 minutes. This reaction was stopped by a suction filtration method using a Wattman GF/B glass filter. The non-specific binding were calculated out from the radioactivity in the presence of 1 mM Gly.

## [3H]SPD binding test

A synapse membrane sample, which had been obtained from a whole brain of rat, was washed three times with a centrifugal operation at 50,000 g for 30 minutes in a 50 mM tris acetate buffer solution (pH 7.4). The pellet was suspended in a 0.32 M sucrose solution, and stored in a frozen state at -80° C. Before the use, the frozen sample was melted at room temperature, and pretreated with a 0.08% triton X-100 at 2° C for 10 minutes. After the pretreatment, a centrifugal cleaning operation of the sample was carried out twice as mentioned above.

In the test, the suspension (protein content: 250 µg) in the above-mentioned buffer was incubated with 20nM [$^3$H]SPD(1202.5 GBq/mmol) at 2° C for 10 minutes. This reaction was stopped by a suction filtration method employing a Wattman GF/B glass filter. The non-specific bindings were calculated out from the radioactivity in the presence of a 10mM SPD.

(2) Inhibition of NMDA-induced tonic convulsion in mice (in vivo)

A test compound was injected intraperitoneally in mice. After 30 minutes from the administration, NMDA was injected intracerebroventicularly, and it was observed whether there was a tonic convulsion. $ED_{50}$ -(mg/kg, i.p.) was calculated out from the inhibition rates.

B. Test results

(1) Binding inhibition at various sites (in vitro)

Table 1

The inhibition potenciesot [$^3H$]-MK-801 binding and [$^3H$]SPD binding

| Compound (Ex. No.) | Inhibition potency of [$^3H$]MK-801 binding (%) ($IC_{50}$, μM) | Inhibition potency of [$^3H$]SPD binding (%) [Inhibition rate (%) in concentration of 10 μg/ml] |
|---|---|---|
| Example 1 | 1.3 | 38.6 |
| " 2 | 5.1 | 80.0 |
| " 3 | 3.7 | 69.0 |
| " 4 | 6.0 | 61.5 |
| " 5 | 0.86 | 57.5 |

As shown in Table 1, inhibition potency of [$^3H$]SPD binding in Compounds 2 to 5 were about 2 to 2.5 times as high as that of Compound 1. The inhibition potency of [$^3H$]MK-801 binding in Compound 5 was about 2 times as high as that of Compound 1. On the other hand, the [$^3H$]Glu, [$^3H$]CPP and [$^3H$]Gly binding inhibition activities of these compounds were not found at a concentration of 10 μg/ml.

(2) Inhibition of NMDA-induced-tonic convulsion in mice (in vitro)

Effects of test compound on NMDA-induced tonic convulsion are shown in Table 2.

Table 2

Inhibition potency of NMDA-induced tonic convulsion in mice

| Compound (Ex. No.) | Inhibition potency of NMDA-induced tonic convulsion ($ED_{50}$; mg/kg, i.p.) |
|---|---|
| Example 1 | 16.4 |
| " 2 | >30 |
| " 3 | >30 |
| " 4 | >30 |
| " 5 | 24.8 |

As shown in Table 2, the inhibition potency of Compound 5 was almost effective as same as that of Compound 1.

From these data, the compounds according to the invention exhibit a high NMDA-antagonistic effect, and these compounds are characterized in that they have a selective antagonistic activity at polyamine sites. Therefore, the compounds are useful as medicines for nerve degeneration diseases.

Examples of the compounds of the invention may include:

1. N-[2-(1-Naphthylamino)ethyl]-N-[2-(2-methylanilino)ethyl]amine
2. N-[2-(1-Naphthylamino)ethyl]-N-[2-(2-methoxyanilino)ethyl]amine
3. N-[2-(1-Naphthylamino)ethyl]-N-[2-(2-nitroanilino)ethyl]amine
4. N-[2-(1-Naphthylamino)ethyl]-N-[2-(2-cyanoanilino)ethyl]amine
5. N-[2-(1-Naphthylamino)ethyl]-N-[2-(2-trifluoromethylanilino)ethyl]amine
6. N-[2-(1-Naphthylamino)ethyl]-N-[2-(2-hydroxyanilino)ethyl]amine
7. N-[2-(1-Naphthylamino)ethyl]-N-[2-(2-methoxycarbonylanilino)ethyl]amine
8. N-[2-(2-Naphthylamino)ethyl]-N-[2-(2-chloroanilino)ethyl]amine
9. N-(2-(1-Naphthylamino)ethyl]-N-[2-(2-naphthylamino)ethyl]amine
10. N-[2-(1-Naphthylamino)ethyl]-N-[2-[(3-cyanopyrid2-yl)amino]ethyl]amine
11. N-[2-(1-Naphthylamino)ethyl]-N-[2-[(3-chloropyrid-2-yl)amino]ethyl]amine
12. N-[2-(1-Naphthylamino)ethyl]-N-[2-[(3-methylpyrid-2-yl)amino]ethyl]amine
13. N-[2-(1-Naphthylamino)ethyl]-N-[2-[(3-methoxypyrid-2-yl)amino]ethyl]amine
14. N-[2-(2-Naphthylamino)ethyl]-N-[2-[(3-chloropyrid-2-yl)amino]ethyl]amine
15. N-[2-(1-Naphthylamino)ethyl]-N-[2-(2-pyrazinylamino)ethyl]amine
16. N-[2-(2-Naphthylamino)ethyl]-N-[2-(2-pyrazinylamino)ethyl]amine
17. N-[2-(2-Naphthylamino)ethyl]-N-[2-(2-pyrimidinylamino)ethyl]amine
18. N-[2-(1-Naphthylamino)ethyl]-N-[2-(3-pyridazinylamino)ethyl]amine
19. N-[2-(2-Naphthylamino)ethyl]-N-[2-(3-pyridazinylamino)ethyl]amine
20. N-[2-(1-Naphthylamino)ethyl]-N-[2-[(1,2,4-triazin-3-yl)amino]ethyl]amine
21. N-[2-(1-Naphthylamino)ethyl]-N-[2-[(1H-indazol-4-yl)amino]ethyl]amine
22. N-[2-(1-Naphthylamino)ethyl]-N-[2-[(1H-indazol-6-yl)amino]ethyl]amine
23. N-[2-(1-Naphthylamino)ethyl]-N-[2-[(1H-benzotriazol-4-yl)amino]ethyl]amine
24. N-[2-(1-Naphthylamino)ethyl]-N-[2-[5,6,7,8-tetrahydronaphth-1-yl)amino]ethyl]amine
25. N-[2-(1-Naphthylamino)ethyl]-N-[2-[(quinol-4-yl)-amino]ethyl]amine
26. N-[2-(1-Naphthylamino)ethyl]-N-[2-[(6-methoxyquinol-8-yl)amino]ethyl]amine

27. N-[2-(1-Naphthylamino)ethyl]-N-[2-[(6-chloroquinol-4-yl)amino]ethyl]amine
28. N-[2-(1-Naphthylamino)ethyl]-N-[2-[(4-methyl-6-methoxyquinol-8-yl)amino]ethyl]amine
29. N-[2-(1-Naphthylamino)ethyl]-N-[2-[(7-methylquinol-4-yl)amino]ethyl]amine
30. N-[2-(1-Naphthylamino)ethyl]-N-[2-[(4-methylquinol-2-yl)amino]ethyl]amine
31. N-[2-(1-Naphthylamino)ethyl]-N-[2-[(isoquinol-1-yl)amino]ethyl]amine
32. N-[2-(1-Naphthylamino)ethyl]-N-[2-[(4-methoxyisoquinol-1-yl)amino]ethyl]amine
33. N-[2-(1-Naphthylamino)ethyl]-N-[2-[(2-methylquinoxalin-3-yl)amino]ethyl]amine
34. N-[2-(1-Naphthylamino)ethyl]-N-[2-[(2-aminoquinazolin-4-yl)amino]ethyl]amine
35. N-[2-(1-Naphthylamino)ethyl]-N-[2-[(6-chloroquinazolin-4-yl)amino]ethyl]amine
36. N-[2-(1-Naphthylamino)ethyl]-N-[2-[(4-methylphthalazin-1-yl)amino]ethyl]amine
37. N-[2-[(1H-indazol-4-yl)amino]ethyl]-N-[2-(2-chloroanilino)ethyl]amine
38. N-[2-(8-Quinolylamino)ethyl]-N-[2-[(1H-indazol-4-yl)amino]ethyl]amine
39. N-[2-(4-Quinolylamino)ethyl]-N-[2-[(1H-indazol-4-yl)amino]ethyl]amine
40. N-[2-(8-Quinolylamino)ethyl]-N-[2-[(1H-indazol-6-yl)amino]ethyl]amine
41. N-[2-(4-Quinolylamino)ethyl]-N-[2-[(1H-indazol-6-yl)amino]ethyl]amine
42. N-[2-(1H-Benzotriazol-4-yl)amino]ethyl]-N-[2-(2-chloroaminimoethyl)]amine
43. N-[2-(8-Quinolylamino)ethyl]-N-[2-[(1H-benzotriazol-4-yl)amino]ethyl]amine
44. N-[2-(4-Quinolylamino)ethyl]-N-[2-[(1H-benzotriazol-4-yl)amino]ethyl]amine
45. N-[2-(2-Chloroanilino)ethyl]-N-[2-[(5,6,7,8-tetrahydronaphth-1-yl)amino]ethyl]amine
46. N-[2-(8-Quinolylamino)ethyl]-N-[2-[(5,6,7,8-tetrahydronaphth-1-yl)amino]ethyl]amine
47. N-[2-(4-Quinolylamino)ethyl]-N-[2-[(5,6,7,8-tetrahydronaphth-1-yl)amino]ethyl]amine
48. N-[2-(8-Quinolylamino)ethyl]-N-[2-(2-methylanilino)ethyl]amine
49. N-[2-(8-Quinolylamino)ethyl]-N-[2-(2-methoxyanilino)ethyl]amine
50. N-[2-(8-Quinolylamino)ethyl]-N-[2-(2-nitroanilino)ethyl]amine
51. N-[2-(8-Quinolylamino)ethyl]-N-[2-(2-cyanoanilino)ethyl]amine
52. N-[2-(8-Quinolylamino)ethyl]-N-[2-(2-hydroxyanilino)ethyl]amine
53. N-[2-(8-Quinolylamino)ethyl]-N-[2-(2-naphthylamino)ethyl]amine
54. N-[2-(8-Quinolylamino)ethyl]-N-[2-(2-pyridylamino)ethyl]amine
55. N-[2-(8-Quinolylamino)ethyl]-N-[2-[(3-cyanopyrid2-yl)amino]ethyl]amine
56. N-[2-(8-Quinolylamino)ethyl]-N-[2-[(3-chloropyrid-2-yl)amino]ethyl]amine
57. N-[2-(8-Quinolylamino)ethyl]-N-[2-[(3-methylpyrid-2-yl)amino]ethyl]amine
58. N-[2-(8-Quinolylamino)ethyl]-N-[2-[(3-methoxypyrid-2-yl)amino]ethyl]amine
59. N-[2-(4-Quinolylamino)ethyl]-N-[2-(2-methylanilino)ethyl]amine
60. N-[2-(4-Quinolylamino)ethyl]-N-[2-(2-methoxyanilino)ethyl]amine
61. N-[2-(4-Quinolylamino)ethyl]-N-[2-(2-nitroanilino)ethyl]amine
62. N-[2-(4-Quinolylamino)ethyl]-N-[2-(2-cyanoanilino)ethyl]amine
63. N-[2-(4-Quinolylamino)ethyl]-N-[2-(2-hydroxyanilino)ethyl]amine
64. N-[2-(4-Quinolylamino)ethyl]-N-[2-(2-quinolylamino)ethyl]amine
65. N,N-Bis[2-(4-quinolylamino)ethyl]amine
66. N-[2-(4-Quinolylamino)ethyl]-N-[2-(8-quinolylamino)ethyl]amine
67. N-[2-(2-Quinolylamino)ethyl]-N-[2-(8-quinolylamino)ethyl]amine
68. N,N-Bis[2-(2-quinolylamino)ethyl]amine
69. N-[2-(1-Isoquinolylamino)ethyl]-N-[2-(8-quinolylamino)ethyl]amine
70. N-[2-(1-Isoquinolylamino)ethyl]-N-[2-(4-quinolylamino)ethyl]amine
71. N-[2-(1-Isoquinolylamino)ethyl]-N-[2-(2-chloroanilino)ethyl]amine
72. N-[2-[(4-Methylphthaladin-1-yl)amino]ethyl]-N-[2-(2-chloroanilino)ethyl]amine
73. N-[2-[(4-Methylphthaladin-1-yl)amino]ethyl]-N-[2-(8-quinolylamino)ethyl]amine
74. N-[2-[(4-Methylphthaladin-1-yl)amino]ethyl]-N-[2-(4-quinolylamino)ethyl]amine
75. N-[2-[(8-Mercaptonaphth-1-yl)amino]ethyl]-N-[2-[(2-methylnaphth-1-yl)amino]ethyl]amine
76. N-[2-[(8-Mercaptonaphth-1-yl)amino]ethyl]-N-[2-[(2-chloronaphth-1-yl)amino]ethyl]amine
77. N-[2-[(8-Mercaptonaphth-1-yl)amino]ethyl]-N-[2-[(8-methylnaphth-1-yl)amino]ethyl]amine
78. N-[2-[(8-Mercaptonaphth-1-yl)amino]ethyl]-N-[2-[(8-chloronaphth-1-yl)amino]ethyl]amine
79. N-[2-[(8-Mercaptonaphth-1-yl)amino]ethyl]-N-[2-[(2,8-dimethylnaphth-1-yl)amino]ethyl]amine
80. N-[2-[(8-Mercaptonaphth-1-yl)amino]ethyl]-N-[2-[(2,8-dichloronaphth-1-yl)amino]ethyl]amine
81. N-[2-[(2-Mercaptonaphth-1-yl)amino]ethyl]-N-[2-[(2-methylnaphth-1-yl)amino]ethyl]amine
82. N-[2-[(2-Mercaptonaphth-1-yl)amino]ethyl]-N-[2-[(2-chloronaphth-1-yl)amino]ethyl]amine
83. N-[2-[(2-Mercaptonaphth-1-yl)amino]ethyl]-N-[2-[(8-methylnaphth-1-yl)amino]ethyl]amine
84. N-[2-[(2-Mercaptonaphth-1-yl)amino]ethyl]-N-[2-[(8-chloronaphth-1-yl)amino]ethyl]amine

85. N-[2-[(2-Mercaptonaphth-1-yl)amino]ethyl]-N-[2-[(2,8-dimethylnaphth-1-yl)amino]ethyl]amine
86. N-[2-[(2-Mercaptonaphth-1-yl)amino]ethyl]-N-[2-[(2,8-dichloronaphth-1-yl)amino]ethyl]amine
87. N-[2-[(1-Mercaptonaphth-2-yl)amino]ethyl]-N-[2-[(2-methylnaphth-1-yl)amino]ethyl]amine
88. N-[2-[(1-Mercaptonaphth-2-yl)amino]ethyl]-N-[2-[(2-chloronaphth-1-yl)amino]ethyl]amine
89. N-[2-[(1-Mercaptonaphth-2-yl)amino]ethyl]-N-[2-[(8-methylnaphth-1-yl)amino]ethyl]amine
90. N-[2-[(1-Mercaptonaphth-2-yl)amino]ethyl]-N-[2-[(8-chloronaphth-1-yl)amino]ethyl]amine
91. N-[2-[(1-Mercaptonaphth-2-yl)amino]ethyl]-N-[2-[(2,8-dimethylnaphth-1-yl)amino]ethyl]amine
92. N-[2-[(1-Mercaptonaphth-2-yl)amino]ethyl]-N-[2-[(2,8-dichloronaphth-1-yl)amino]ethyl]amine
93. N-[2-[(2-Mercaptonaphth-3-yl)amino]ethyl]-N-[2-[(2,8-dimethylnaphth-1-yl)amino]ethyl]amine
94. N-[2-[(8-Hydroxyiminomethylnaphth-1-yl)amino]-ethyl]-N-[2-[(2,8-dichloronaphth-1-yl)amino]-ethyl]amine
95. N-[2-[(2-Hydroxyiminomethylnaphth-1-yl)amino]-ethyl]-N-[2-[(2,8-dichloronaphth-1-yl)amino]-ethyl]amine
96. N-[2-[(8-Acetamidonaphth-1-yl)amino]ethyl]-N-[2-[(2,8-dichloronaphth-1-yl)amino]ethyl]amine
97. N-[2-[(8-Nitronaphth-1-yl)amino]ethyl]-N-[2-[(2,8-dichloronaphth-1-yl)amino]ethyl]amine
98. N-[2-[(8-Guanidinonaphth-1-yl)amino]ethyl]-N-[2-[(2,8-dichloronaphth-1-yl)amino]ethyl]amine
99. N-[2-[(8-Methanesulfinylnaphth-1-yl)amino]ethyl]-N-[2-[(2,8-dichloronaphth-1-yl)amino]ethyl]-amine
100. N-[2-[(8-Methanesulfonylnaphth-1-yl)amino]ethyl]-N-[2-[(2,8-dichloronaphth-1-yl)amino]ethyl]-amine
101. N-[2-[(8-Sulfamoylnaphth-1-yl)amino]ethyl]-N-[2-[(2,8-dichloronaphth-1-yl)amino]ethyl]amine
102. N-[2-[(8-Carbamoylnaphth-1-yl)amino]ethyl]-N-[2-[(2,8-dichloronaphth-1-yl)amino]ethyl]amine
103. N-[2-[[8(N-Hydroxycarbamoyl)naphth-1-yl]amino]-ethyl]-N-[2-[(2,8-dichloronaphth-1-yl)amino]-ethyl]amine
104. N-[2-[[8-(N-Hydroxyacetamido)naphth-1-yl]amino]-ethyl]-N-[2-[(2,8-dichloronaphth-1-yl)amino]-ethyl]amine

Reference Examples

Preparations of compounds (VIII) and (IX) of the following formulas will be mentioned below.

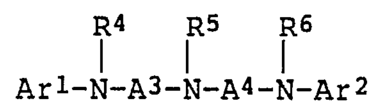

(VIII)

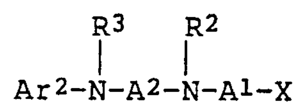

(IX)

Reference Example 1

Preparation of N-(1-naphthylamino)ethyl-2-(1-naphthyl-amino)acetamide dihydrochloride

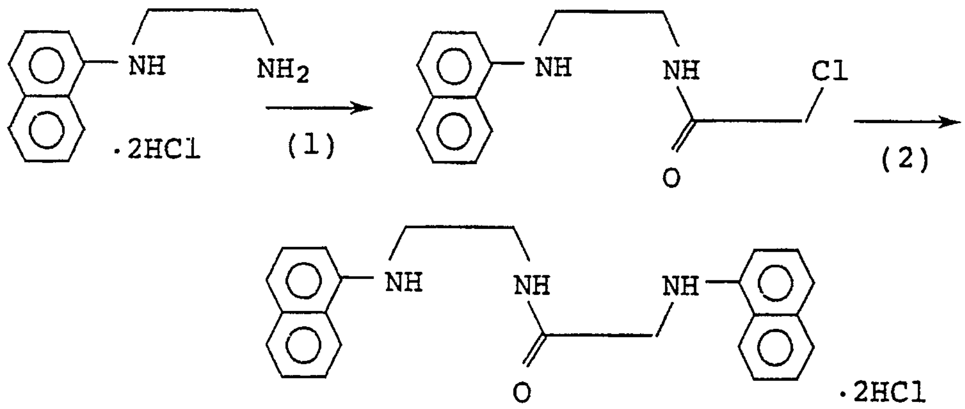

(1) A solution of 12 g (0.11 mol) of chloroacetyl chloride in 24 ml of tetrahydrofuran was dropwise added to a mixture of 25 g (0.097 mol) of N-1-naphthylethylenediamine dihydrochloride, 39 g (0.39 mol) of triethylamine and 25G ml of tetrahydrofuran, at a temperature of at most 10°C under cooling with ice water and under stirring. After the completion of this addition, the reaction mixture was stirred at room temperature for 2 hours, and the crystals thus precipitated were removed by filtration. The filtrate was concentrated under a reduced pressure, and the residue was purified by a column chromatography (250 g of silica gel; chloroform as eluant) to obtain 18.1 g of [N-(1-naphthylamino)ethyl]-2-chloractamide. Yield: 70.9%.

(2) A mixture of 2.65 g (0.01 mol) of [N-(1-naphthylamino)ethyl]-2-chloracetamide, 2.1 g (0.015 mol) of 1-naphthylamine, 2.1 g (0.015 mol) of anhydrous potassium carbonate and 27 ml of n-butyl alcohol was stirred under reflux for 7 hours. The reaction mixture wad cooled, then poured into water, and extracted with ethyl acetate. The organic layer was twice washed with water, dried over sodium sulfate, and concentrated under a reduced pressure. The resulting crude oily product was purified by a column chromatography (250 g of silica gel; chloroform as eluant), and then treated with 13.5% hydrogen chloride/isopropyl alcohol to give 650 mg of the title compound. mp. 105°C (decomposition).

Reference Example 2

Preparation of 3-[(1-naphthylamino)ethylamino]-N-(1-naphthyl)propionamide dihydrochloride

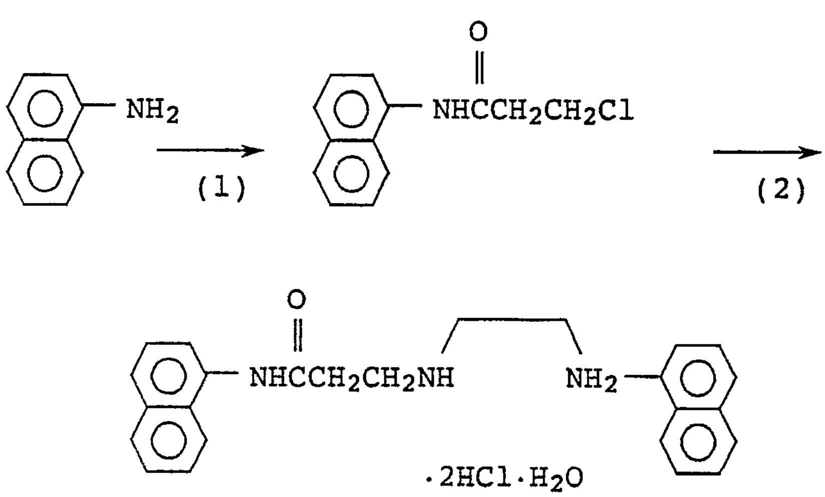

(1) A solution of 27.9 g (0.22 mol) of 3-chloropropionyl chloride in 55.8 ml of tetrahydrofuran was added dropwise to a mixture of 28.6 g (0.2 mol) of 1-naphthylamine, 30.4 g (0.3 mol) of triethylamine and 286 ml of tetrahydrofuran, at an internal temperature of at most 5°C under cooling with ice water and under stirring. After the completion of this addition, the reaction mixture was stirred at room temperature for 24

hours. The crystals thus precipitated were removed by filtration, and the filtrate was concentrated under a reduced pressure.

The resulting residue was admixed with 500 ml of ethyl acetate and washed with water, and a separating operation was carried out. The organic layer was washed with 200 ml of a 10% aqueous hydrochloric acid solution, and a separating operation was effected. Then the organic layer was washed with water, thereafter washed with a dilute aqueous sodium hydroxide solution, and dried over magnesium sulfate. The ethyl acetate was distilled off under a reduced pressure. The crystals thus formed were washed with n-hexane, and separated by filtration to give 34 g of N-(1-naphthyl)-3-chloropropionamide. Yield: 72.7%.

(2) A mixture of 4.7 g (0.02 mol) of N-(1-naphthyl)-3-chloropropionamide, 5.2 g (0.02 mol) of N-(1-naphthyl)ethylenediamine dihydrochloride, 8.3 g (0.06 mol) of anhydrous potassium carbonate, 0.3 g (2 mmol) of potassium iodide and 47 ml of n-butyl alcohol was refluxed for 8 hours. The reaction mixture was cooled, poured into water, and extracted with ethyl acetate. The organic layer was twice washed with water, dried over magnesium sulfate, and concentrated under a reduced pressure. The residue was purified by a column chromatography (250 g of silica gel; chloroform as eluant), so that 3 g (39.1%) of a free product were obtained. 0.8 g of said free product was treated with 13.5% hydrogen chloride/isopropyl alcohol to give 0.86 g of the title compound in the form of dihydrochloride monohydrate. mp. 227-228 °C (decomposition).

Reference Example 3

Preparation of N-[2-(1-naphthylamino)ethyl]-2-(8-quinolylamino)-acetamide

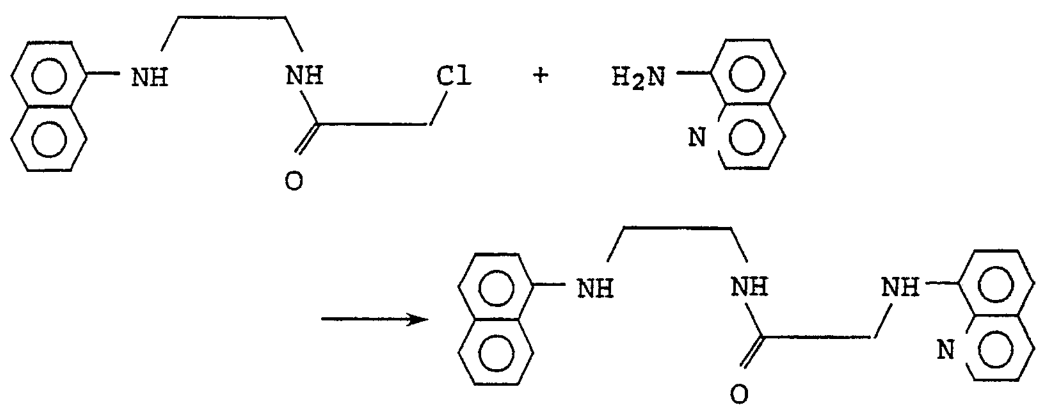

A mixture of 4 g (15.1 mmol) of [N-[2-(1-naphthylamino)ethyl]-2-chloroacetamide, 2.6 g (18.1 mmol) of 8-aminoquinoline, 2.5 g (18.1 mmol) of anhydrous potassium carbonate and 26 ml of n-butyl alcohol was refluxed for 7.5 hours. Then the n-butyl alcohol was distilled off, and the residue was heated to 120 °C for 6 hours. The reaction mixture was subjected to an extraction operation, wherein use was made of 200 ml of water and 400 ml of ethyl acetate. The organic layer was twice washed with 200 ml of water, and dried over magnesium sulfate. The solvent was distilled off under a reduced pressure, and 6.3 g of the residue was purified by a column chromatography (250 g of silica gel; ethyl acetate/n-hexane as eluant) to obtain 1.3 g of the title compound. Yield: 23.2%; mp. 150-151 °C.

Reference Example 4

Preparation of [2-(1-naphthylamino)ethylamino]-N-(1-naphthyl)acetamide monohydrochloride monohydrate

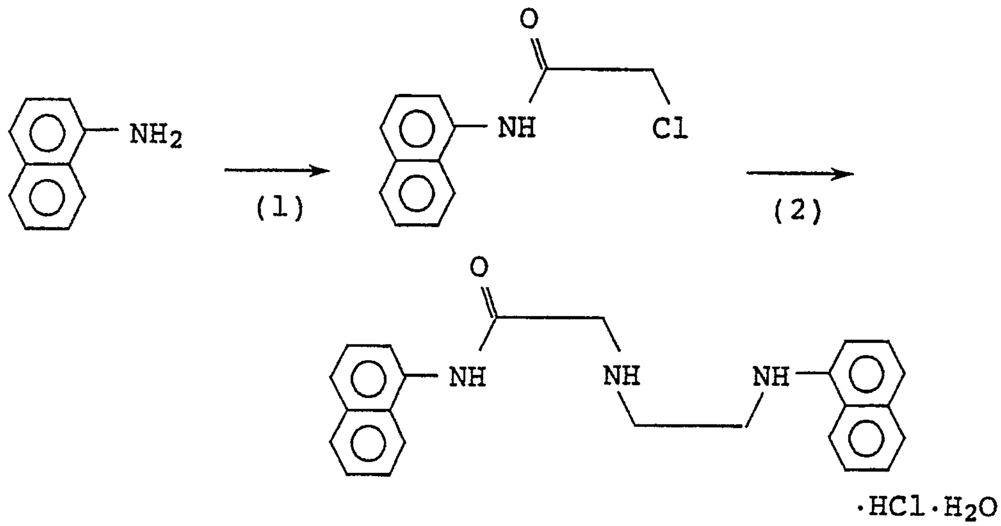


(1) A solution of 22.5 g (0.2 mol) of chloroacetyl chloride in 22.5 ml of tetrahydrofuran was added dropwise to a solution cf 14.3 g (0.1 mol) of 1-naphthylamine in 143 ml of tetrahydrofuran at room temperature under stirring. The reaction mixture was stirred at room temperature for 4.5 hours. Then the tetrahydrofuran was distilled off, and the crystals thus formed were washed with ethyl acetate to give 13.5 g (61.4%) of N-(1-naphthyl)-2-chloroacetamide. mp. 161-162° C.

(2) A mixture of 2.3 g (8.87 mmol) of N-(1-naphthyl)ethylenediamine dihydrochloride, 1.9 g (8.87 mmol) of N-(1-naphthyl)-2-chloroacetamide, 3.7 g (26.6 mmol) of anhydrous potassium carbonate, 0.15 g (0.89 mmol) of potassium iodide and 46 ml of n-butyl alcohol was refluxed for 5.5 hours. The reaction mixture was filtered when it was still hot. The filtrate was distilled under a reduced pressure to remove the solvent therefrom, so that 4 g of an oily residue was obtained. The residue was purified by a column chromatography (250 g of silica gel; ethyl acetate/n-hexane(1:1) as eluant) to give 1.9 g (59.5%z) of a free crystalline product. This product was treated with 9.6% hydrogen chloride/isopropyl alcohol, and then recrystallized from a mixture of 50 ml of methyl alcohol and 100 ml of isopropyl alcohol to give 1.5 g (39.1%) of the title compound. mp. 206-208° C.

Reference Example 5

Preparation of [2-(1-naphthylamino)ethylamino]-N-(5-quinoxalyl)-acetamide dihydrochloride

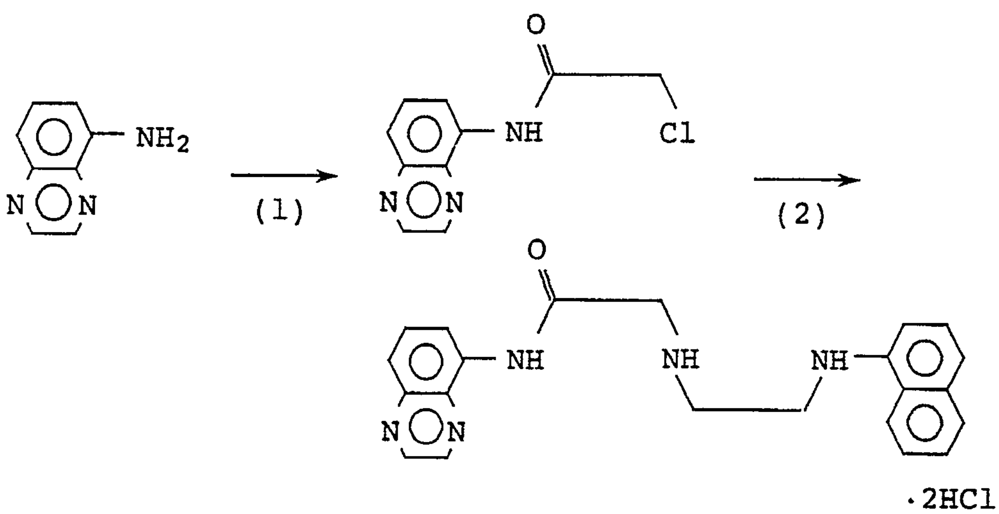


(1) A solution of 3.4 g (0.03 mol) of chloroacetyl chloride in 6.8 ml of tetrahydrofuran was dropwise

added to a mixture of 2.9 g (0.02 mol) of 5-aminoquinoxaline, 4 g (0.04 mol) of triethylamine and 29 ml of tetrahydrofuran under cooling with ice water. The reaction mixture was kept at such a low temperature for 4.5 hours. Then the solvent was distilled off under a reduced pressure, and the residue was purified by a column chromatography (250 g of silica gel; chloroform as eluant) to give 1.6 g (36.1%) of N-(5-quinoxalyl)-2-chloroacetamide. mp. 163-165° C.

(2) A mixture of 1.6 g (7.22 mmol) of N-(5-quinoxalyl)-2-chloroacetamide, 1.9 g (7.22 mmol) of N-(1-naphthyl)ethylenediamine dihydrochloride, 3 g (21.7 mmol) of anhydrous potassium carbonate, 0.12 g (0.72 mmol) of potassium iodide and 16 ml of dimethylformamide was kept at an internal temperature of 90 to 100° C for 9 hours. Then the reaction mixture was poured into water, and the resulting precipitated crystals were separated by filtration, so that 3.4 g of a crude product was obtained as crystals. The crude product was purified by a column chromatography (250 g of silica gel; chloroform as eluant) to give 1.1 g (41%) of a free product. A portion of the free product was treated with 13.5% hydrogen chloride/isopropyl alcohol to obtain the title compound. mp. 215° C.

Reference Example 6

Preparation of [2-(1-naphthylamino)ethylamino-N-(3-quinolyl)acetamide dihydrochloride monohydrate

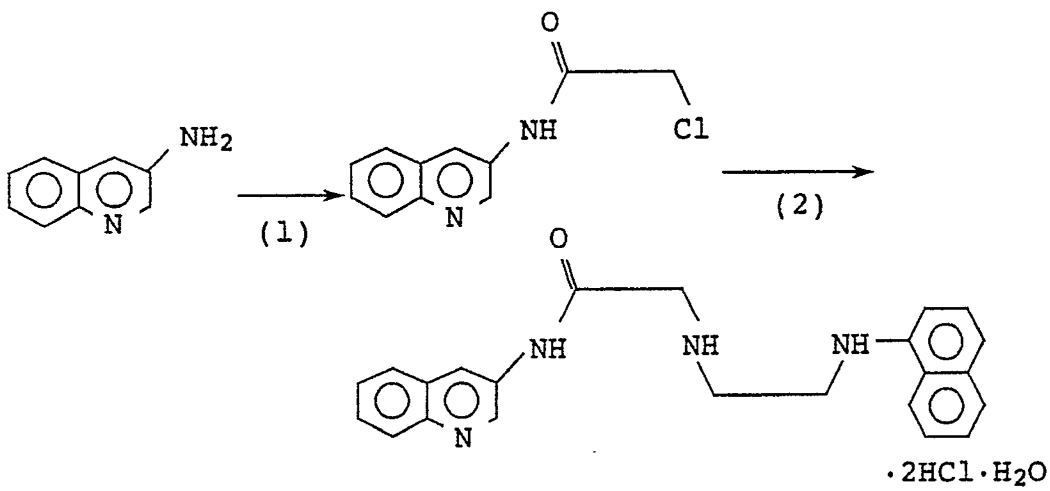


(1) A solution of 11.3 g (0.1 mol) of chloroacetyl chloride in 22.6 ml of tetrahydrofuran was dropwise added to a mixture of 9.7 g (0.0673 mol) of 3-aminoquinoline, 13.6 g (0.135 mol) of triethylamine and 97 ml of tetrahydrofuran. After the completion of the addition, the reaction mixture was stirred at room temperature for 4 hours, and then filtered. The filtrate was distilled, and the resultant residue was admixed with ethyl acetate and water to effect an extraction operation. The organic layer was washed with a saturated aqueous sodium bicarbonate solution and then with water, and dried over magnesium sulfate. The solvent was distilled off under a reduced pressure to give N-(3-quinolyl)-2-chloroacetamide.

(2) A mixture of 11 g (0.05 mol) of N-(3-quinolyl)-2-chloroacetamide, 14.2 g (0.055 mol) of N-(1-naphthyl)-ethylenediamine dihydrochloride, 22.8 g (0.165 mol) of anhydrous potassium carbonate, 0.83 g (0.05 mol) of potassium iodide and 110 ml of dimethylformamide was stirred for 4 hours. The reaction mixture was poured into water, and extracted with ethyl acetate. The organic layer was three times washed with water, then filtered through celite, and dried over magnesium sulfate. The solvent was distilled off under a reduced pressure to give 20.4 g of a crude oily product. The crude product was purified by a column chromatography (250 g of silica gel; chloroform as eluant) to obtain 12.2 g (65.9%) of a caramel product. The last-mentioned product was treated with 13.5% hydrogen chloride/isopropyl alcohol, so that the title compound was obtained. mp. 187° C (decomposition).

Reference Example 7

Preparation of [2-(1-naphthylamino)ethylamino]-N-(5-quinolyl)acetamide dihydrochloride monohydrate

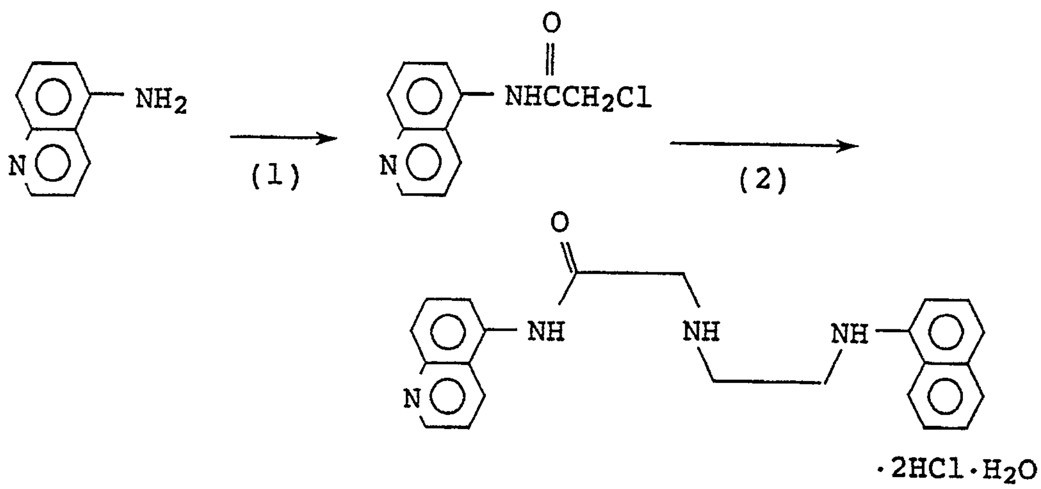

(1) A solution of 17.6 g (0.156 mol) of chloroacetyl chloride in 35.2 ml of tetrahydrofuran was dropwise added to a mixture of 15 g (0.104 mol) of 5-aminoquinoline, 21.1 g (0.208 mol) of triethylamine and 150 ml of tetrahydrofuran, under cooling with ice water. The reaction mixture was then stirred at room temperature for 4 hours, and thereafter poured into water. The resulting mixture was acidified with hydrochloric acid, and extracted with ethyl acetate. The mixture was filtered through celite, and the filtrate was twice washed with water, dried over magnesium sulfate, and subjected to a distillation operation under a reduced pressure to remove the solvent therefrom. 11.2 g (48.8%) of N-(5-quinolyl)-2-chloroacetamide was obtained.

(2) A mixture of 10.6 g (0.048 mol) of N-(5-quinolyl)-2-chloroacetamide, 12.4 g (0.048 mol) of N-(1-naphthyl)ethylenediamine dihydrochloride, 19.9 g (0.144 mol) of anhydrous potassium carbonate, 0.8 g (0.0048 mol) of potassium iodide and 106 ml of dimethylformamide was heated to an internal temperature of 90 to 100 °C for 7.5 hours. Then the reaction mixture was poured into ice water, and extracted with ethyl acetate. The extraction product was filtered through celite, and the filtrate was washed with water and dried over magnesium sulfate. The solvent was distilled off under a reduced pressure to give 11.3 g of a crude oily product.

The aqueous layer was again extracted with chloroform, and the resultant extraction product was treated in a conventional manner to obtain 2.2 g of a crude oily product.

The both crude oily products were mixed, and the resulting mixture was purified by a column chromatography (250 g of silica gel; chloroform as eluant) to give 4 g (22.5%) of a free product, which was then treated with 13.5% hydrogen chloride/isopropyl alcohol, so that the title compound was obtained. mp. 235-237 °C (decomposition).

<u>Reference Example 8</u>

<u>Preparation of 2-[2-(1-naphthylamino)ethylamino]-N-(1-naphthyl)propionamide monohydrate</u>

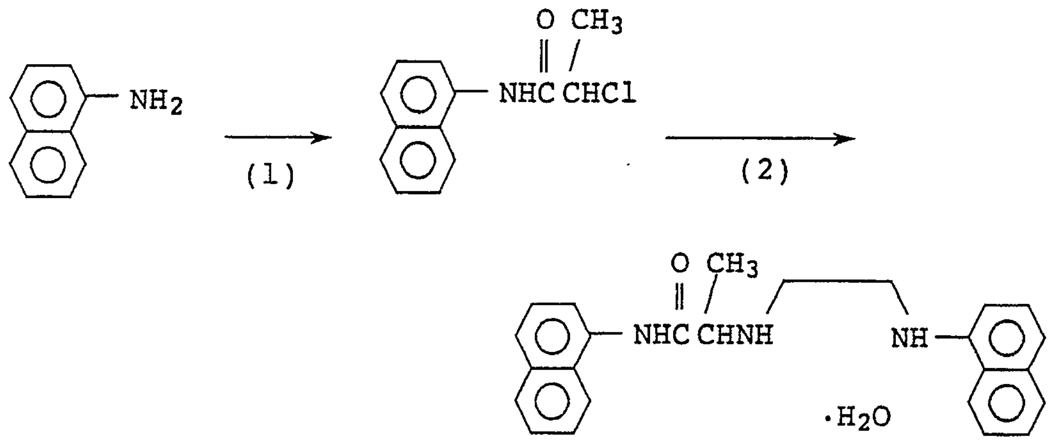


(1) A solution of 12.7 g (0.1 mol) of 2-chloropropionyl chloride in 12.7 ml of tetrahydrofuran was dropwise added to a solution of 12.9 g (0.09 mol) of 1-naphthylamine in 129 ml of tetrahydrofuran under cooling with ice water. Then the reaction mixture was stirred at the same temperature for 45 minutes, and thereafter stirred at room temperature for 1 hour. The crystals thus precipitated were washed with ether, and filtered off. The filtrate was concentrated under a reduced pressure. The resulting crystalline residue was isolated by filtration with ether, so that 10.5 g (49.9%) of N-(1-naphthyl)2-chloropropionamide was obtained. mp. 143-144°C.

(2) A mixture of 3.5 g (0.015 mol) of N-(1-naphthyl)-2-chloropropionamide, 3.5 g (0.014 mol) of N-(1-naphthyl)ethylenediamine dihydrochloride, 8.3 g (0.06 mol) of anhydrous potassium carbonate, 1 g (6 mmol) of potassium iodide and 70 ml of acetonitrile was refluxed for 8.5 hours. The reaction mixture was poured into water, and extracted with ethyl acetate. The resultant extract was washed with water, and dried over magnesium sulfate. The solvent was distilled off to give 6.2 g of a crude crystalline product, which were then recrystallized from 300 ml of isopropyl alcohol. 3.2 g (61.8%) of the title compound was obtained. mp. 154-155°C.

Reference Example 9

Preparation of N-[(1-naphthylamino)ethyl]-2-(1-naphthylamino)propionamide dihydrochloride

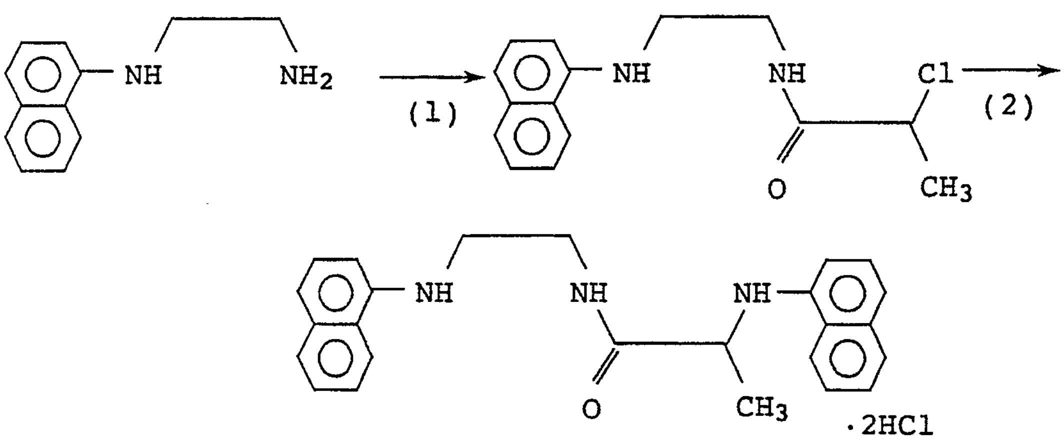


(1) A solution of 12.7 g (0.1 mol) of 2-chloropropionyl chloride in 12.7 ml of tetrahydrofuran was dropwise added to a mixture of 23.3 g (0.09 mol) of N-1-naphthyl-ethylenediamine dihydrochloride, 36.4 g (0.36 mol) of triethylamine and 233 ml of tetrahydrofuran, under cooling with ice water. Then the reaction mixture was stirred at the same temperature for 45 minutes, and thereafter stirred at room temperature for 1 hour. The crystals thus precipitated were filtered off, and the filtrate was concentrated under a reduced pressure to give 27.4 g of a crude oily product, which were then purified by a column

chromatography (250 g of silica gel; chloroform as eluant). 8.9 g (35.7%) of N-[(1-naphthylamino)ethyl]-2-chloropropionamide was obtained. mp. 80-82 °C.

(2) A mixture of 2.8 g (0.01 mol) of N-[(1-naphthylamino)ethyl]-2-chloropropionamide, 2.9 g (0.02 mol) of 1-naphthylamine, 2.8 9 (0.02 mol) of anhydrous potassium carbonate, 0.3 g (2 mmol) of potassium iodide and 29 ml of dimethylformamide was heated to an internal temperature of 90 to 100 °C for 23 hours. Then the reaction mixture was poured into water, and extracted with ethyl acetate. The organic layer was twice washed with water, then washed with a saturated aqueous sodium chloride solution, and dried over magnesium sulfate. The solvent was distilled off under a reduced pressure to obtain 6.0 g of a crude oily product, which were then purified by a column chromatography (250 g of silica gel; n-hexane (initially) and ethyl acetate as eluant), so that 0.7 g (18.3%) of a free product was obtained. A portion of the free product was treated with 13.5% hydrogen chloride/isopropyl alcohol to obtain the title compound. mp. 147 °C (decomposition).

Example 1

Preparation of bis-[2-(8-quinolylamino)ethyl]amine trihydrochloride monohydrate

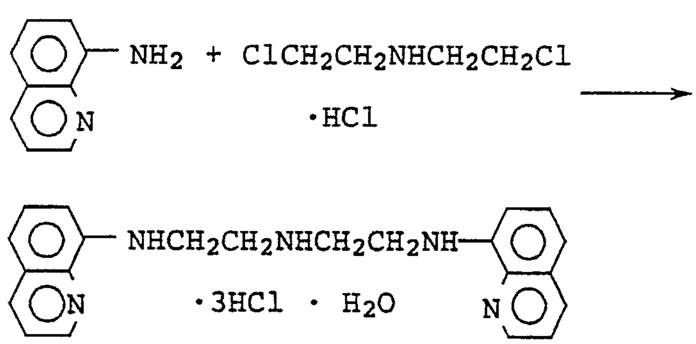

A mixture of 15 g (0.1 mol) of 8-amino-quinoline, 17.8 g (0.1 mol) of bis(2-chloroethyl)amine hydrochloride, 21.2 g (0.2 mol) of sodium carbonate and 100 ml of n-butyl alcohol was refluxed under stirring for 35.5 hours. Then the reaction mixture was poured into 200 ml of water, and the aqueous layer was alkalinized with potassium hydroxide, and extracted with 200 ml of ethyl acetate to obtain 25.4 g of a crude oily product, which were then purified by a silica gel column chromatography, so that 1.4 g of a free product (yield: 3.9%) was obtained. The free product was treated with 9.7% hydrogen chloride/isopropyl alcohol, and recrystallized from methanol to give 1.3 g of the title compound. Yield: 2.7% mp; 214-218 °C.

This compound is known, and can also be prepared by a method in Acta. Chem. Scand. 18, 1 (1964).

Example 2

Preparation of bis-[2-(1-naphthylamino)ethyl]amine dihydrochloride

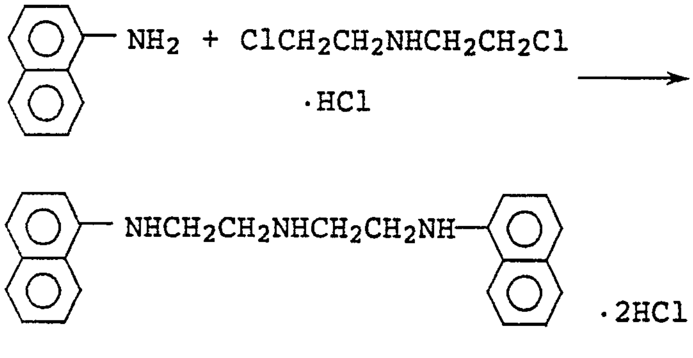

A mixture of 60 g (0.42 mol) of α-naphthylamine, 37.5 g (0.21 mol) of bis(2-chloroethyl)amine hydrochloride, 22.3 g (0.21 mol) of sodium carbanate and 180 ml of n-butyl alcohol was refluxed under stirring for 13 hours. Then the reaction solution was poured into 400 ml of water, and the aqueous layer was admixed with potassium hydroxide to adjust the this layer to a pH of 11 to 12. Then the aqueous layer was extracted with 300 ml of ethyl acetate, and again extracted with 200 ml of ethyl acetate. The combined organic layers were dried over magnesium sulfate, and the solvent was distilled off. The residue was purified by a silica gel chromatography to give 7.74 g (yield: 10.4%) of a free product, which was then treated with 9.7% hydrogen chloride/isopropyl alcohol. 10 g of a crude crystalline product was obtained. The crude product was recrystallized from 900 ml of methanol to give 4.5 g of the title compound. mp 223 - 225 ° C.

Example 3

Preparation of bis-[2-(5, 6, 7, 8-tetrahydro-1-naphthylamino)ethyl]amine trihydrochloride

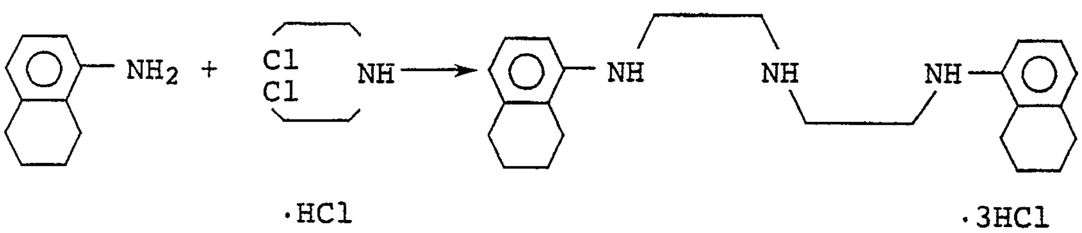

A mixture of 3.6 g (0.02 mol) of bis(2-chloroethyl)amine hydrochloride, 14.7 g (0.1 mol) of 1-amino-5,6,7,8-tetrahydronaphthalene, 8.3 g (0.06 mol) of anhydrous potassium carbonate, 6.6 g (0.04 mol) of potassium iodide and 60 ml of n-butyl alcohol was reflexed for 15 hours. Then the reaction mixture was poured into water, and extracted with ethyl acetate. The organic layer was twice washed with water, and dried over magnesium sulfate. The solvent was distilled off under a reduced pressure. The residue was purified by a column chromatography (250 g of cilica gel; $CHCl_3$ as eluant) to give 3.5 g (48.1%) of an oily product, which was then treated with 13.5% hydrogen chloride/isopropyl alcohol. 4.4 g of the title compound was obtained. mp. 210-215 ° C.

Example 4

Preparation of N-[2-(1-naphthylamino)ethyl]-N-[3-(1-naphthylamino)propyl]amine dihydrochloride

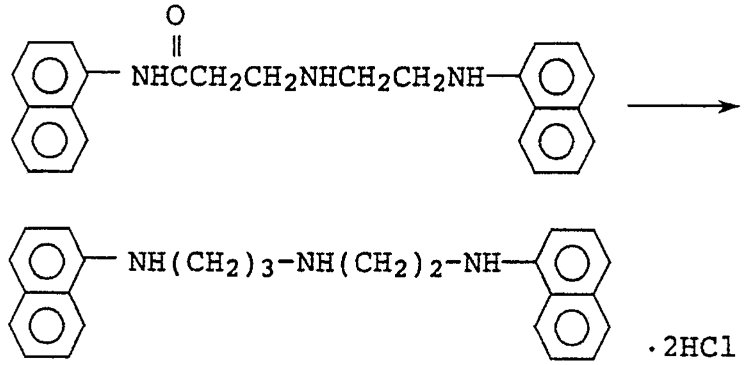

10 ml of tetrahydrofuran was supplied to 0.44 g (0.012 mol) of lithium aluminium hydride under an atmosphere of $N_2$ gas. To the resulting mixture was dropwise added a solution of 2.2 g (5.74 mmol) of 3-(1-naphthylamino)ethylamino-N-(1-naphthyl)-propionamide in 10 ml of tetrahydrofuran under stirring at room

temperature. Then the reaction mixture was stirred for 3 hours, admixed with 0.44 ml of water, and then admixed with 1.3 ml of a 28% aqueous NaOH solution. The reaction mixture was stirred for 30 minutes, and filtered through celite. The filtrate was concentrated under a reduced pressure, and the residue was purified by a column chromatography (250 g of silica gel; chloroform as eluant) to give 0.6 g of a free product as oil, which was then treated with 13.5% hydrogen chloride/isopropyl alcohol. The title compound was obtained in an amount of 0.63 g. Yield: 24.8%; mp. 229-231 °C.

Example 5

Preparation of bis-[2-(4-chloro-1-naphthylamino)ethyl]-amine trihydrochloride

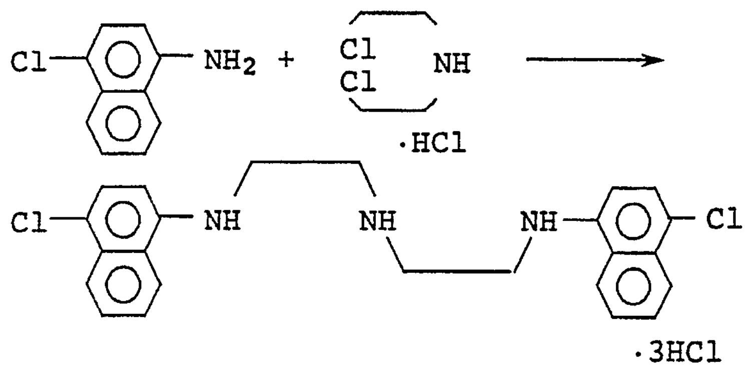

8.9 g (0.05 mol) of 1-amino-4-chloronaphthalene was heated, and admixed with 1.8 g (0.01 mol) of bis-(2-chloroethyl)amine hydrochloride at an internal temperature of 130 °C. The reaction mixture became hard, and then the mixture was admixed with 4 g (0.05 mol) of pyridine, kept at the same temperature for 2 hours, and thereafter admixed with 1.8 g (0.01 mol) of bis-(2-chloroethyl)amine hydrochloride. Then the reaction mixture was kept under a warm condition for further 1 hour, and extracted with ethyl acetate. The resultant extract was washed with an aqueous sodium hydroxide solution, then washed twice with water, and dried over magnesium sulfate. The solvent was distilled off under a reduced pressure, and the residue was purified by a column chromatography (250 g of silica gel; chloroform as eluant) to give 1.5 g of an oily product, which was then treated with 13.5% hydrogen chloride/isopropyl alcohol. 1.5 g of the title compound was obtained. mp. 230-231 °C.

Example 6

Preparation of N-[2-(1-naphthylamino)ethyl]-N-[2-(8-quinolylamino)ethyl]amine trihydrochloride monohydrate

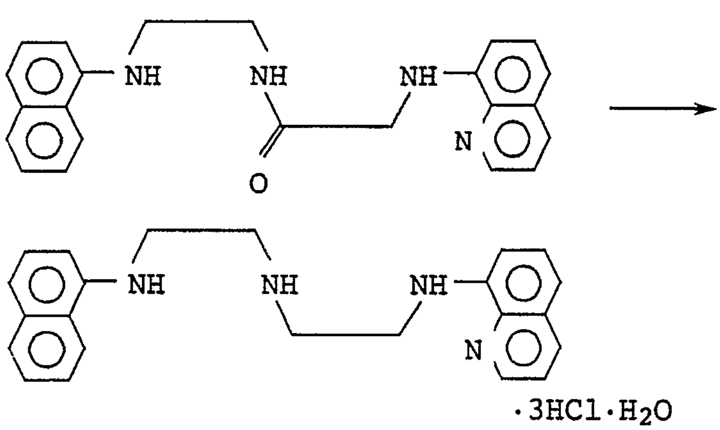

A solution of 1.2 g (3.24 mmol) of N-[2-(1-naphthylamino)ethyl]-2-(8-quinolylamino)actamide in 20 ml of tetrahydrofuran was added dropwise to a mixture of 246 mg (6.48 mmol) of lithium aluminium hydride and 10 ml of tetrahydrofuran under stirring in a nitrogen gas atmosphere at room temperature. Then the reaction mixture was kept at the same temperature for 2 hours, and thereafter admixed with 123 mg (3.24 mmol) of lithium aluminium hydride. The reaction mixture was kept at the same temperature for 4.5 hours, and then admixed with 0.37 ml of water and thereafter with 1.5 ml of a 28% aqueous NaOH solution. Then the reaction mixture was filtered through celite, and the filtrate was distilled under a reduced pressure. The residue was purified by a column chromatography (250 g of silica gel; chloroform as eluant) to give 0.6 g (52.5%) of an oily product, which was then treated with 13.5% hydrogen chloride/isopropyl alcohol. 0.36 g of the title compound was obtained. mp. 130-132 (decomposition).

Example 7

Preparation of N-[2-(ethyl-1-naphthylamino)ethyl]-N-[2-(1-naphthylamino)ethyl]amine oxalate 1/2-hydrate

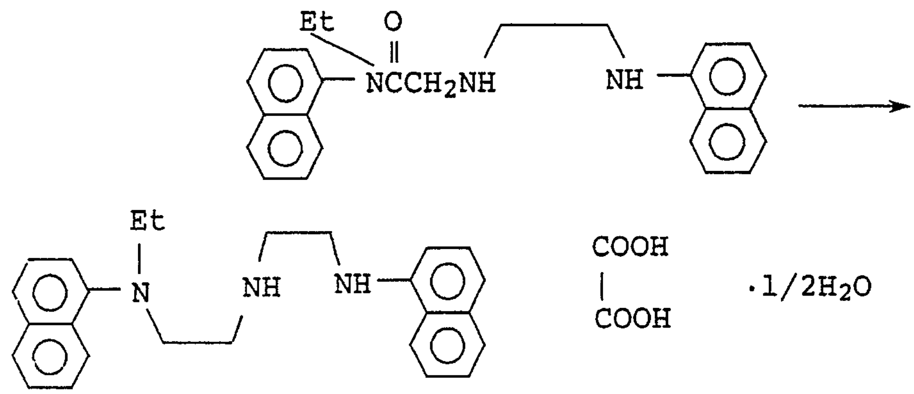

A solution of 4.2 g (0.01 mol) of 2-[(1-naphthylamino)ethylamino]-N-(ethyl-1-naphthyl)acetamide in 42 ml of tetrahydrofuran was added dropwise to a mixture of 0.8 g (0.021 mol) of lithium aluminium hydride and 16 ml of tetrahydrofuran under stirring under a nitrogen gas atmosphere at room temperature. Then the reaction mixture was kept at the same temperature for 1.5 hours. After the completion of the reaction, 4 ml of a 10% aqueous sodium hydroxide solution was dropwise added to the reaction mixture under cooling with ice water. Then the reaction mixture was stirred at room temperature for 30 minutes, and thereafter filtered through celite. The filtrate was distilled under a reduced pressure to give 4.1 g of a crude oily product, which were then purified by a column chromatography (250 g of silica gel; chloroform as eluant), so that 0.6 g (14.8%) of an oily product was obtained. The last-mentioned oily product was treated with 0.2 g (1.56 mmol) of oxalic acid to give 0.6 g of the title compound.

Elementary analysis (as $C_{28}H_{31}N_3O_4.1/2H_2O$):

Calc.: C 69.69; H 6.68; N 8.71

Found: C 69.88; H 6.88; N 8.55

Example 8

Preparation of N-[2-(1-naphthylamino)ethyl]-N-[[1-methyl-2-(1-naphthylamino)]ethyl]amine hydrochloride

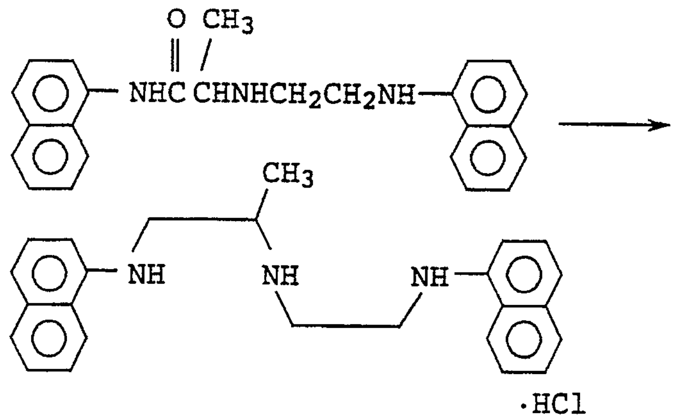

A solution of 2.5 g (6.52 mmol) of 2-[2-(1-naphthylamino)ethylamino]-N-(1-naphthyl)propionamide in 25 ml of tetrahydrofuran was dropwise added to a mixture of 0.5 g (13 mmol) of lithium aluminium hydride and 12.5 ml of tetrahydrofuran under stirring under a nitrogen gas atmosphere at room temperature. Then the reaction solution was stirred at room temperature for 3.5 hours, and thereafter cooled with ice water. 2.5 ml of a 10% aqueous NaOH solution were dropwise added to the reaction mixture, which was then stirred at room temperature 30 minutes, and filtered through celite. The filtrate was concentrated under a reduced pressure to give 2.7 g of a crude oily product, which was then purified by a column chromatography (250 g of silica gel; chloroform as eluant), so that 1.7 g (70.6%) of a crystalline product were obtained. The crystalline product was treated with 13.5% hydrogen chloride/isopropyl alcohol to give 1.5 g of the title compound. mp. 212-214° C.

Example 9

Preparation of N-[(1-naphthylamino)ethyl]-N-[2-(1-naphtylamino)propyl]amine

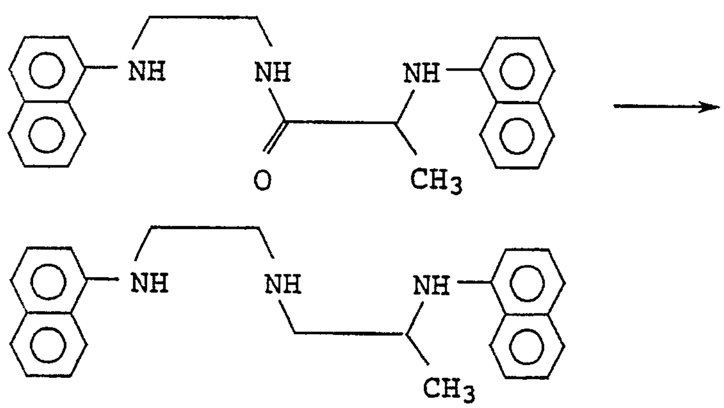

A solution of 0.6 g (1.56 mmol) of N-[(1-naphthylamino)ethyl]-2-(1-naphthylamino)propionamide in 5 ml of tetrahydrofuran was added dropwise to a mixture of 180 mg (4.69 mmol) of lithium aluminium hydride and 3 ml of tetrahydrofuran under stirring at room temperature under a nitrogen gas atmosphere. Then the reaction mixture was stirred for 3 hours. 1 ml of a 10% aqueous NaOH solution was added dropwise to the reaction mixture under cooling with ice water. The reaction mixture was then stirred at room temperature for 30 minutes, and filtered through celite to give 0.6 g of a crude oily product, which was thereafter purified by a column chromatography (200 g of silica gel; chloroform as eluant). 0.5 g (86.5%) of the title compound was obtained.

NMR (δ $CDCl_3$): 1.33 3H(d, 6Hz), 1.65

1H(broad), 2.84-3.03 2H(m), 3.09 2H(t, 6Hz), 3.28-3.42 2H(m), 3.75-3.89 1H(m), 4.7 1H(broad), 5.0 1H(broad), 6.59 1H(d, 7Hz), 6.67 1H(d, 7Hz), 7.2-7.49 8H(m), 7.73-7.87 4H(m).

Example 10

Preparation of N-[2-(1-naphthylamino)ethyl]-N-[2-(2-quinolylamino)ethyl]amine dihydrochloride monohydrate

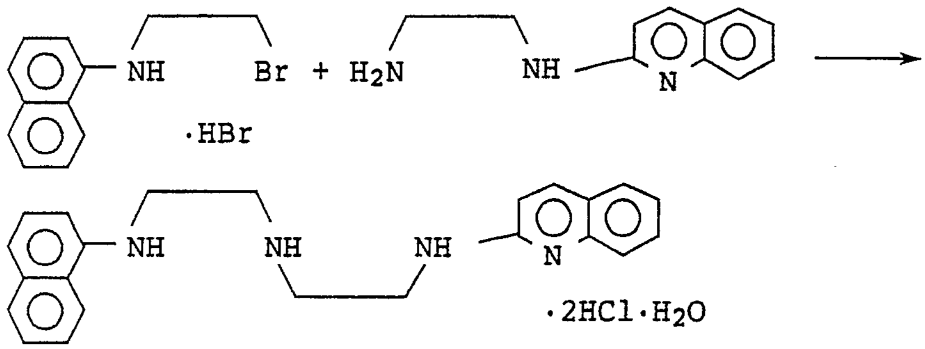

A mixture of 5 g (15 mmol) of N-(2-bromoethyl)-1-naphthylamine hydrobromide, 2.8 g (15 mmol) of N-(2-quinolyl)ethylenediamine, 6.3 g (45 mmol) of anhydrous potassium carbonate and 50 ml of acetonitrile was refluxed for 11 hours. The reaction mixture was poured into water, and extracted with ethyl acetate. The organic layer was twice washed with water, and dried over magnesium sulfate. The solvent was distilled off under a reduced pressure to give 5.3 g of a crude oily product, which was then purified by a column chromatography (250 g of silica gel; chloroform as eluant), and thereafter treated with 9.5% hydrogen chloride/isopropyl alcohol to give 1.0 g (15%) of the title compound.

Elementary Analysis (as $C_{23}H_{28}Cl_2N_4O$)

Calc.: C 61.74; H 6.31; N 12.52

Found: C 62.44; H 6.22; N 12.43

Example 11

Preparation of N-[2-(1-naphthylamino)ethyl]-N-[2-(2-pyrimidinylamino)ethyl]amine oxalate

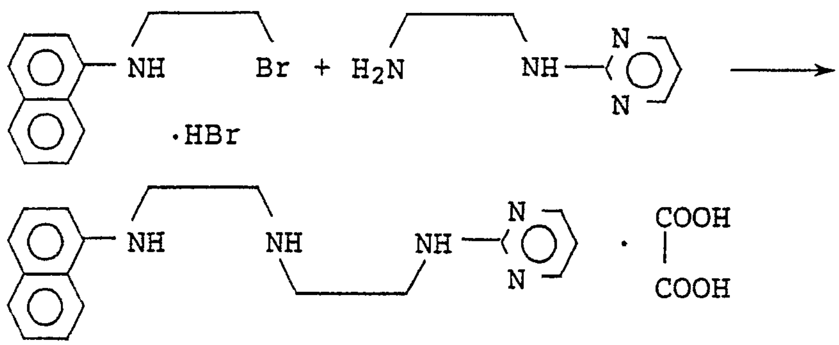

The reaction procedures of Example 10 were repeated, except that 2.1 g (15 mmol) of N-(2-pyrimidinyl)ethylenediamine was used instead of N-(2-quinolyl)ethylenediamine. 1.3 g of the title compound was obtained. Yield: 21.8%; mp. 201 °C (decomposition).

Example 12

Preparation of N-[2-(1-naphthylamino)ethyl]-N-[2-anilinoethyl]amine dihydrochloride

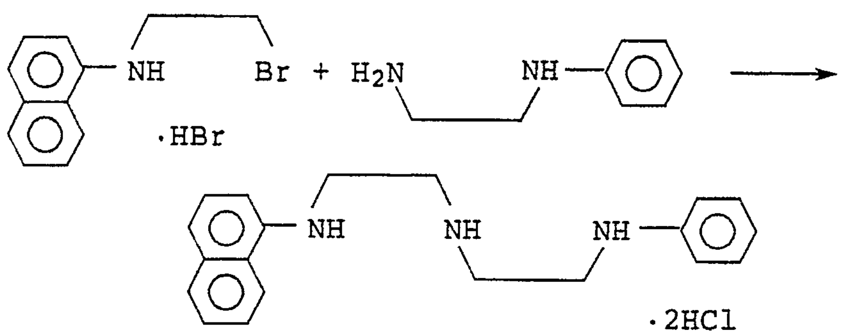

The reaction procedures of Example 10 were repeated, except that 2.1 g (15 mmol) of N-phenylethylenediamine was used instead of N-(2-quinolyl)-ethylenediamine. 1.3 g of the title compound was obtained. Yield: 22.9%; mp. 186-187 °C.

Example 13

Preparation of N-[2-(1-naphthylamino)ethyl]-N-[2-(4-chloroanilino)ethyl]amine trihydrochloride

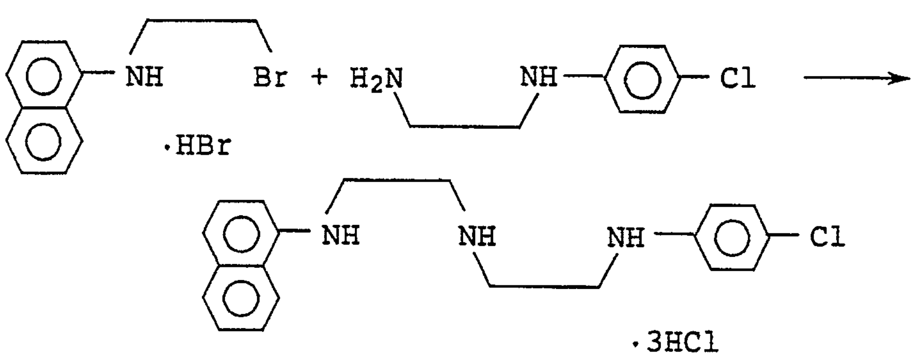

The reaction procedures of Example 10 were repeated, except that 3.4 9 (20 mmol) of N-(4-chlorophenyl)ethylenediamine was used instead of N-(2-quinolyl)ethylenediamine. 2.7 g of the title compound was obtained. Yield: 29.4%; mp. 183-185 ° C.

Example 14

Preparation of N-[2-(1-naphthylamino)ethyl]-N-[2-(3-chloro-anilino)ethyl]amine trihydrochloride

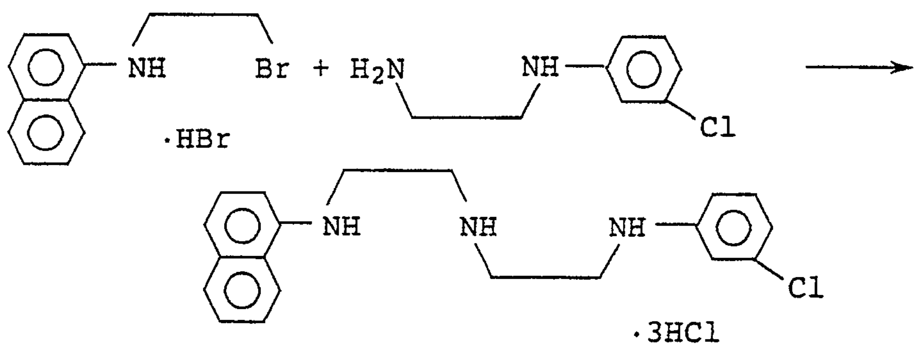

The reaction procedures of Example 10 were repeated, except that 3.4 g (20 mmol) of N-(3-chlorophenyl)ethylenediamine was used instead of N-(2-quinolyl)ethylenediamine. 1.6 g of the title compound was obtained. Yield: 17.4%.

Elementary analysis (as $C_{20}H_{25}Cl_4N_3$):

Calc.: C 53.47; H 5.61; N 9.35

Found: C 53.87; H 6.06; N 9.02

Example 15

Preparation of N-[2-(1-naphthylamino)ethyl]-N-[2-(2-chloro-anilino)ethyl]amine dihydrochloride

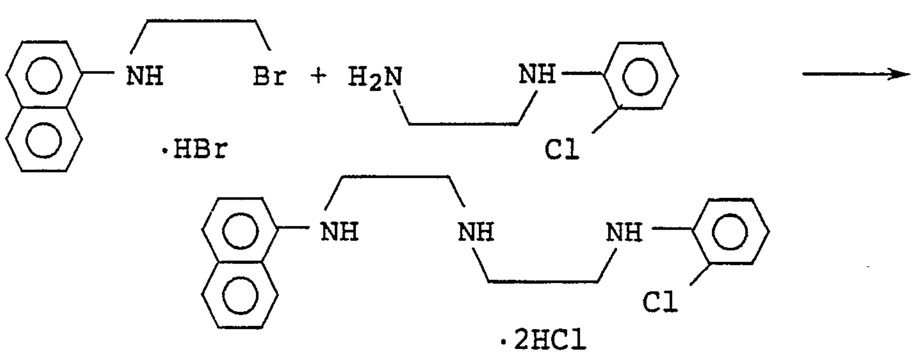

The reaction procedures of Example 10 were repeated, except that 2.4 g (14 mmol) of N-(2-chlorophenyl)ethylenediamine was used instead of N-(2-quinolyl)ethylenediamine. 1.9 g of the title compound was obtained. Yield: 32.9%; mp. 225-230 ° C.

Example 16

Preparation of N-[2-(1-naphthylamino)ethyl]-N-[2-(diphenylamino)ethyl]amine monohydrochloride monohydrate

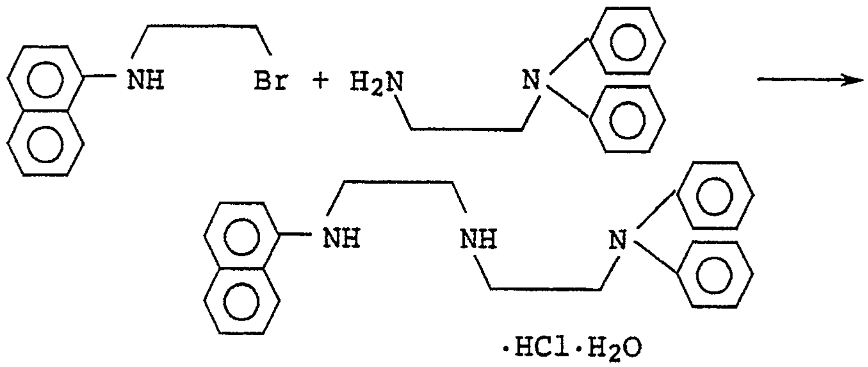

The reaction procedures of Example 10 were repeated, except that 1.6 g (7.54 mmol) of N,N-diphenylethylenediamine was used instead of N-(2-quinolyl)-ethylenediamine. 0.45 g of the title compound was obtained. Yield: 12.2%; mp. 196-198° C.

Example 17

Preparation of bis-[2-(1-naphthylamino)ethyl]methylamine trihydrochloride

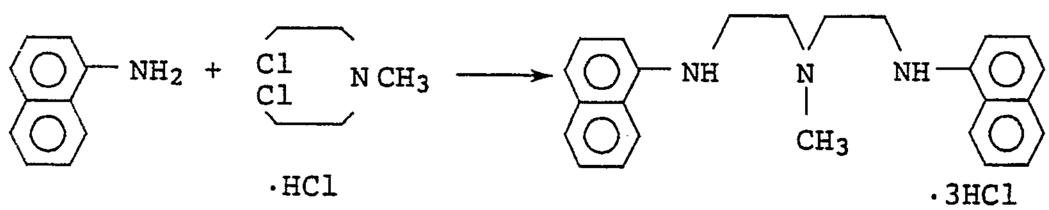

A mixture of 5 g (o.026 mol) of mechlorethamine hydrochloride, 74.4 g (0.52 mol) of 1-naphthylamine, 7.2 g (0.052 mol) of anhydrous potassium carbonate and 81 ml of n-butyl alcohol was refluxed for 37 hours. Then the reaction mixture was poured into water, and extracted with ethyl acetate. The organic layer was washed with water, dried over magnesium sulfate, and distilled under a reduced pressure to remove the solvent therefrom. The resultant crude product was purified by a column chromatography (250 g of silica gel; n-hexane(initially) and ethyl acetate as eluant) to give 3.1 g (32.3%) of an oily product, which was then treated with 9.5% hydrogen chloride/isopropyl alcohol. 3.4 g of the title compound was obtained. mp. 197° C (decomposition).

Example 18

Preparation of N-[2-(1-naphthylamino)ethyl]-N-[2-(4-methoxyanilino)ethyl]amine hydrochloride

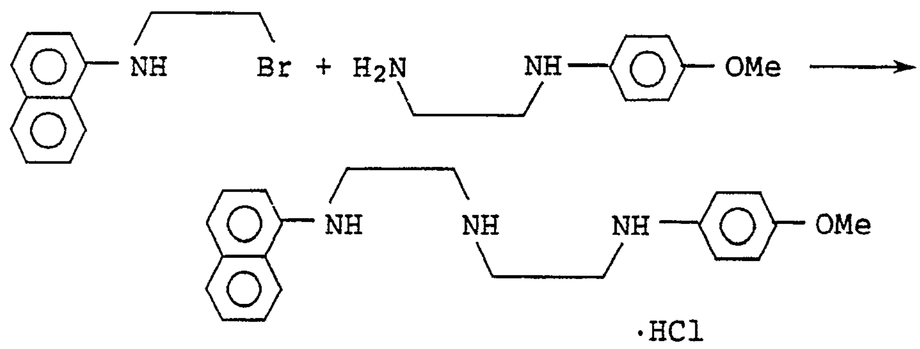

A mixture of 2.8 g (16.9 mmol) of N-(4-methoxyphenyl)ethylenediamine, 5 g of KF-celite, 5.6 g (16.9 mmol) of N-(2-bromoethyl)-1-naphthylamine hydrobromide and 100 ml of acetonitrile was refluxed for 6 hours. The celite was filtered off, and the filtrate was concentrated and then purified by a column chromatography(silica gel; ethyl acetate/ethanol/triethylamine (20/2/1) as eluant). Thereafter, a hydrogen chloride/isopropyl alcohol treatment was effected to give 720 mg of the title compound. Yield: 13%; mp. 95-98 °C.

Example 19

Preparation of N-[2-(1-naphthylamino)ethyl]-N-[2-(3-cyano-anilino)ethyl]amine hydrochloride

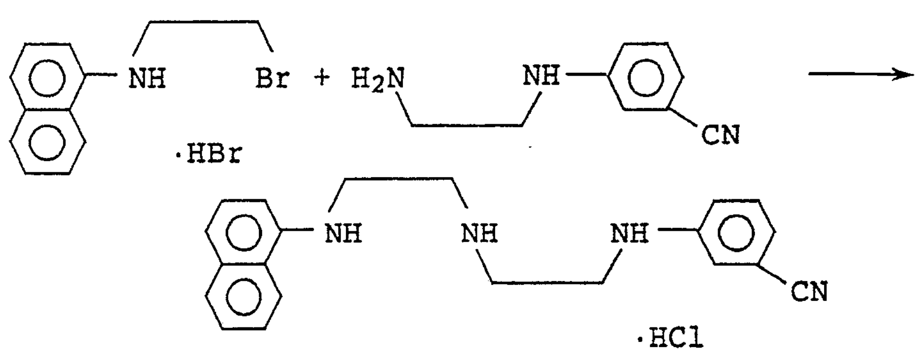

The reaction procedures of Example 18 were repeated, except that 3.2 g (20 mmol) of N-(3-cyanophenyl)ethylenediamine was used instead of N-(4-methoxyphenyl)ethylenediamine. The title compound was obtained. mp. 115-120 °C.

Example 20

Preparation of N-[2-(1-naphthylamino)ethyl]-N-[2-(2-pyridylamino)ethyl]amine hydrochloride

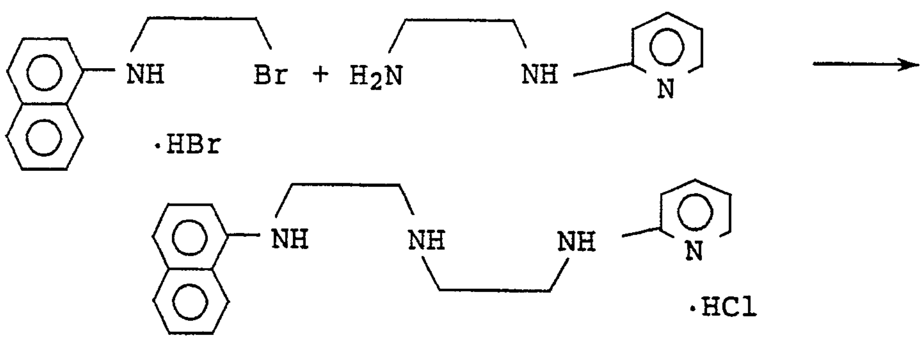

The reaction procedures of Example 18 were repeated, except that 2.6 g (15 mmol) of N-(2-pyridyl)-ethylenediamine was used instead of N-(4-methoxyphenyl)ethylenediamine. The title compound was obtained. mp. 164-167 °C.

Example 21

Preparation of bis-[2-(8-amino-1-naphthylamino)ethyl]-amine hydrochloride

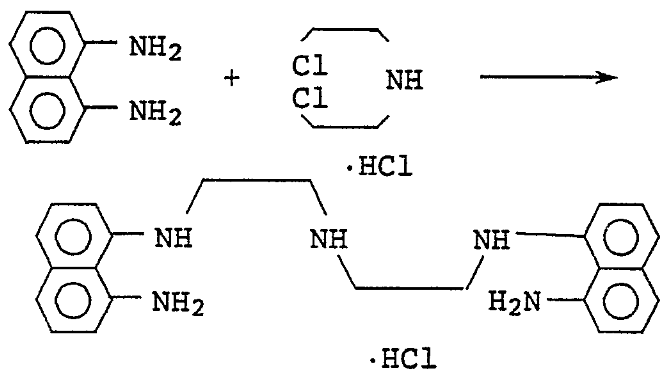

A mixture of 3.95 g (25 mmol) of 1,8-diaminonaphthalene, 1 g (6.25 mmol) of bis-(2-chloroethyl)amine hydrochloride, 5 g of KF-celite and 20 ml of acetonitrile was reflexed for 10 hours. The insoluble matters were filtered off, and the filtrate was concentrated under a reduced pressure. The resulting residue was purified by a column chromatography, and then treated with hydrogen chloride/isopropyl alcohol to give the title compound. mp. 150-153 °C.

Example 22

Preparation of N-[2-(8-quinolylamino)ethyl]-N-[2-(3,4-dichloro-anilino)ethyl]amine hydrochloride

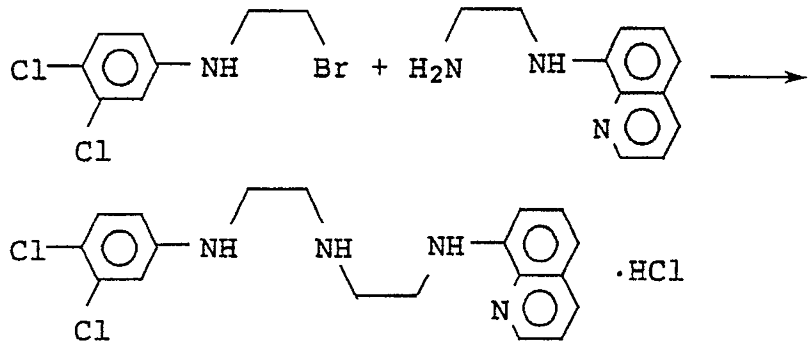

The reaction procedures of Example 18 were repeated, except that 1.5 g (8 mmol) of N-(8-quinolyl)-ethylenediamine was used instead of N-(4-methoxyphenyl)-ethylenediamine, and also except that 1.7 g (5 mmol) of N-(2-bromoethyl)-3,4-dichloro-aniline hydrobromide was employed instead of N-(2-bromoethyl)-naphthylamine hydrochloride. The title compound was obtained. mp. 110-113 ° C.

## Claims

1. A triamine derivative of the formula (I)

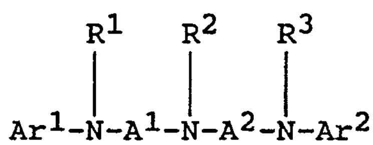

wherein $Ar^1$ is an unsubstituted or substituted aryl group; an unsubstituted or substituted 6-memberd heterocyclic aromatic group containing 1 to 3 nitrogen atoms; an unsubstituted or substituted bicyclic heterocyclic aromatic group having a 5-membered heterocyclic ring condensed with a benzene ring and containing 1 to 3 nitrogen atoms; an unsubstituted or substituted bicylic heterocylic aromatic group having a 5-membered heterocylic ring condensed with a benzene ring and containing one sulfur atom and optionally one nitrogen atom; an unsubstituted or substituted bicyclic heterocyclic aromatic group having a 5-membered heterocyclic ring condensed with a benzene ring and containing one oxygen atom and optionally one nitrogen atom; an unsubstituted or substituted bicyclic carbocyclic aromatic group having a 5-or 6-membered hydrated carbocyclic ring condensed with a benzene ring; or an unsubstituted or substituted bicyclic heterocyclic aromatic group having a 6-membered heterocyclic ring condensed with a benzene ring and containing 1 or 2 nitrogen atoms;

$Ar^2$ is an unsubstituted or substituted naphthyl group; an unsubstituted or substituted bicyclic heterocyclic aromatic group having a 5-membered heterocyclic ring condensed with a benzene ring and containing 1 to 3 nitrogen atoms; an unsubstituted or substituted bicyclic heterocyclic aromatic group having a 5-membered heterocyclic ring condensed with a benzene ring and containing one sulfur atom and optionally one nitrogen atom; an unsubstituted or substituted bicyclic heterocyclic aromatic group having a 5-membered heterocyclic ring condensed with a benzene ring and containing one oxygen atom and optionally one nitrogen atom; an unsubstituted or substituted bicyclic carbocyclic aromatic group having a 5-or 6-membered hydrated carbocylic ring condensed with a benzene ring; or an unsubstituted or substituted bicyclic heterocyclic aromatic group having a 6-membered heterocyclic ring condensed with a benzene ring and containing 1 or 2 nitrogen atoms;

$A^1$ and $^2$ are the same or different and each is a lower alkylene group optionally substituted by one or more oxo groups; and

$R^1$, $R^2$ and $R^3$ are the same or different and each is hydrogen, lower alkyl, aryl, aryl-alkyl, aryl-alkyloxycarbonyl, alkyloxycarbonyl or acyl;

or an acid addition salt thereof,

for use in the prevention or treatment of the prognostic symptoms assocaited with cerebral

apoplexy or cerebral infarction.

2. Use of a compound as defined in Claim 1 in the manufacture of a medicament for use in the prevention or the treatment of prognostic symptoms associated with cerebral apoplexy or cerebral infarction.

3. A triamine derivative of the formula (I) as depicted in Claim 1 wherein $Ar^1$ is an unsubstituted or substituted aryl group; an unsubstituted or substituted 6-membered heterocyclic aromatic group containing 1 to 3 nitrogen atoms; an unsubstituted or substituted bicyclic heterocyclic aromatic group having a 5-membered heterocyclic ring condensed with a benzene ring and containing 1 to 3 nitrogen atoms; an unsubstituted or substituted bicyclic carbocyclic aromatic group having a 5- or 6-membered hydrated carbocyclic ring condensed with a benzene ring; or an unsubstituted or substituted bicyclic heterocyclic aromatic group having a 6-membered heterocyclic ring condensed with a benzene ring and containing 1 or 2 nitrogen atoms;

$Ar^2$ is an unsubstituted or substituted naphthyl group; an unsubstituted or substituted bicyclic heterocyclic aromatic group having a 5-membered heterocyclic ring condensed with a benzene ring and containing 1 to 3 nitrogen atoms; an unsubstituted or substituted bicyclic carbocyclic aromatic group having a 5- or 6-membered hydrated carbocyclic ring condensed with a benzene ring; or an unsubstituted or substituted bicyclic heterocyclic aromatic group having a 6-membered heterocyclic ring condensed with a benzene ring and containing 1 or 2 nitrogen atoms;

one of $A^1$ and $A^2$ is an ethylene group optionally substituted by an alkyl group and the other of $A^1$ and $A^2$ is a lower alkylene group;

$R^1$ and $R^3$ are the ame or different and each is hydrogen, lower alkyl, aryl, aryl-alkyl, aryl-alkyloxycarbonyl, alkyloxycarbonyl or acyl; and

$R^2$ is hydrogen, lower alkyl, aryl-alkyl, aryl-alkyloxycarbonyl, alkyloxycarbonyl or acyl, or an acid addition salt thereof.

With the proviso that, when $Ar^1$ is an 8-quinolyl group, both $A^1$ and $A^2$ are an ethylene group, and each of $R^1$, $R^2$ and $R^3$ is hydrogen, then $Ar^2$ it not an 8-quinolyl group;

4. A compound according to claim 3, wherein $R^1$, $R^2$, and $R^3$ are hydrogen.

5. A compound according to claim 4 wherein $R^1$, $R^2$, and $R^3$ are hydrogen; and $A^1$, and $A^2$ are ethylene.

6. A compound according to claim 5 wherein $Ar^1$ is an unsubstituted or substituted aryl group; an unsubstituted or substituted 6-membered heterocyclic aromatic group containing 1 to 3 nitrogen atoms; an unsubstituted or substituted bicyclic carbocyclic aromatic group having a 6-membered hydrated carbocyclic ring condensed with a benzene ring; or an unsubstituted or substituted bicyclic heterocyelic aromatic group having a 6-membered heterocyclic ring condensed with a benzene ring and containing 1 or 2 nitrogen atoms;

$Ar^2$ represents an unsubstituted or substituted naphthyl group; an unsubstituted or substituted bicyclic carbocyclic aromatic group having a 6-membered hydrated carbocyclic ring condensed with a benzene ring; or an unsubstituted or substituted bicyclic heterocyclic aromatic group having a 6-membered heterocyclic ring condensed with a benene ring and containing 1 or 2 nitrogen atoms.

7. A process for the preparation of a compound as claimed in any one of claims 3 to 6, which comprises:
(a), where $Ar^1$ and $Ar^2$ are the same and $R^1$ and $R^3$ are the same, reacting a compound or formula (II)

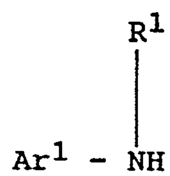

(II)

in which $Ar^1$ and $R^1$ are as defined in claim 3 with a compound of formula (III):

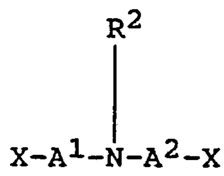

(III)

in which $A^1$, $A^2$ and $R^2$ are as defined in in claim 3 and X is halogen, alkyl- or aryl-sulfonyloxy, hydroxy or lower alkoxy;

(b) where $Ar^1$ is the same as $Ar^2$, reacting a compound of formula (IV):

$Ar^1$-$X^2$ (IV)

in which $Ar^1$ is as defined in claim 3 and $X^2$ is halogen, with a compound of formula (V):

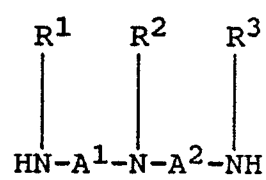

(V)

in which $A^1$, $A^2$, $R^1$, $R^2$ and $R^3$ are as defined in claim 3;

(c) reacting a compound of formula (VI):

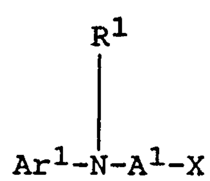

(VI)

in which $Ar^1$, $A^1$ and $R^1$ are as defined in claim 3 and X is as hereinbefore defined, with a compound of formula (VII)

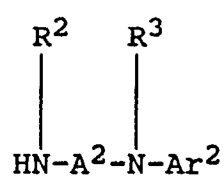

(VII)

in which $Ar^2$, $A^2$, $R^2$ and $R^3$ are as defined in claim 3;

(d) where are least one of $A^1$ and $A^2$ is a lower alkylene group, reducing a compound of formula (VIII)

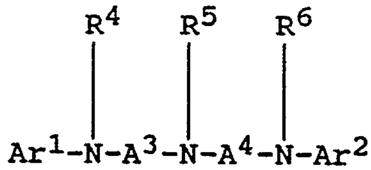

(VIII)

in which $Ar^1$ and $Ar^2$ are as defined in claim 3, $A^3$ and $A^4$, which are the same or different, are each lower alkylene substituted by one or two oxo groups, and $R^4$, $R^5$ and $R^6$ which are the same or different, are each hydrogen or lower alkyl; or

(e) reacting a compound of formula (IX)

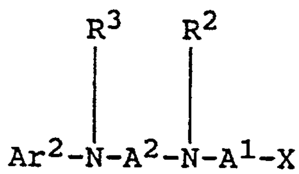

(IX)

in which $Ar^2$, $A^1$, $A^2$, $R^2$ and $R^3$ are as defined in claim 3 and X is as hereinbefore defined with a compound of formula (II) as hereinbefore defined;

and, where appropriate, converting the product thus obtained to an acid addition salt thereof.

**8.** A pharmaceutical composition which comprises a compound according to any one of claims 3 to 6 in association with a pharmaceutically acceptable diluent or carrier.

European Patent Office

# EUROPEAN SEARCH REPORT

Application Number

EP 91301417.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 66, no. 7, February 13, 1967 Columbus, Ohio, USA P.H. NIELSEN et al. "Studies of chelates with heterocyclic ligands.II. Ionic nickel(II) and copper (II) chelates with 8-aminoquinoline and some of its derivatives" page 3 270, abstract-no. 34 404n & Acta Chem. Scand., 1966, 20(4), 1 113-21 -- | 3 | C 07 C 211/58<br>C 07 C 211/57<br>C 07 C 217/84<br>C 07 C 255/58<br>C 07 C 237/10<br>C 07 C 237/04<br>C 07 D 215/40<br>C 07 D 215/38<br>C 07 D 239/42<br>C 07 D 213/74<br>A 61 K 31/135<br>A 61 K 31/16<br>A 61 K 31/47<br>A 61 K 31/44<br>A 61 K 31/505 |
| A | CHEMICAL ABSTRACTS, vol. 70, no. 1, January 6, 1969 Columbus, Ohio, USA E.F. ELSLAGER et al. "Synthetic amebicides.VIII. 7,7-Iminobis(alkylenimino)bis (benz(c)acridines) and congenersic 9-aminoacridines, 12-aminobenz(a)acridines, 12-amino(b)acridines and 4-aminoquinolines" page 352, abstract-no. 3 812x & J. Heterocycl. Chem. 1968, 5(6), 599-607 -- | 3,7 | **TECHNICAL FIELDS SEARCHED (Int. Cl.5)**<br>C 07 C 211/00<br>C 07 C 237/00<br>C 07 C 217/00<br>C 07 D 215/00<br>C 07 D 233/00<br>C 07 D 213/00<br>C 07 D 307/00<br>C 07 D 333/00 |
| A | CH - A - 482 643 (RICHTER GEDEON VEGYESZETI GYAR RT) * Claims * ---- | 1,3,7 | |

The present search report has been drawn up for all claims

| Place of search | Date of completion of the search | Examiner |
|---|---|---|
| VIENNA | 03-06-1991 | KÖRBER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)